(19) Europäisches Patentamt
European Patent Office
Office européen des brevets

(11) Numéro de publication : **0 300 908 B1**

(12) # FASCICULE DE BREVET EUROPEEN

(45) Date de publication du fascicule du brevet : **18.03.92 Bulletin 92/12**

(51) Int. Cl.$^5$ : **C07D 311/58,** C07D 405/06, C07D 405/14, A61K 31/445, A61K 31/495

(21) Numéro de dépôt : **88401890.4**

(22) Date de dépôt : **21.07.88**

(54) **Nouveaux dérivés du benzopyranne, leur préparation et les compositions pharmaceutiques qui les contiennent.**

Le dossier contient des informations techniques présentées postérieurement au dépôt de la demande et ne figurant pas dans le présent fascicule.

(30) Priorité : **23.07.87 FR 8710453**

(43) Date de publication de la demande : **25.01.89 Bulletin 89/04**

(45) Mention de la délivrance du brevet : **18.03.92 Bulletin 92/12**

(84) Etats contractants désignés : **AT BE CH DE ES FR GB GR IT LI LU NL SE**

(56) Documents cités :
**EP-A- 0 114 374**
**EP-A- 0 157 267**

(73) Titulaire : **RHONE-POULENC SANTE**
**20, avenue Raymond Aron**
**F-92160 Antony (FR)**

(72) Inventeur : **Hardy, Jean-Claude**
**1, Passage des Griottes**
**F-95800 Cergy St. Christophe (FR)**
Inventeur : **Renault, Christian**
**61, rue des Mallets**
**F- 95150 Taverny (FR)**

(74) Mandataire : **Savina, Jacques et al**
**RHONE-POULENC INTERSERVICES Service Brevets Pharma 25, quai Paul Doumer**
**F-92408 Courbevoie Cédex (FR)**

EP 0 300 908 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention concerne de nouveaux dérivés du benzopyranne de formule générale :

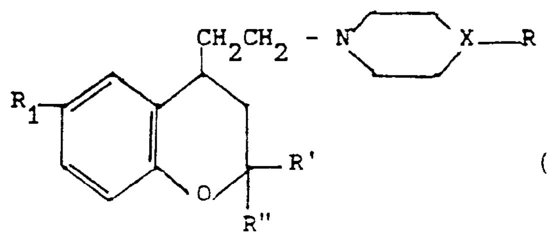

(I)

leurs sels, leur préparation et les compositions pharmaceutiques qui les contiennent.

Dans la demande de brevet EP-A-157267 ont été décrits des dérivés du benzopyranne actifs comme hypotenseurs.

Dans la demande de brevet allemand 3 300 004 ont été décrits des dérivés de l'aminométhyl-4 benzopyranne actifs comme hypotenseurs et relaxants musculaires, et répondant à la formule :

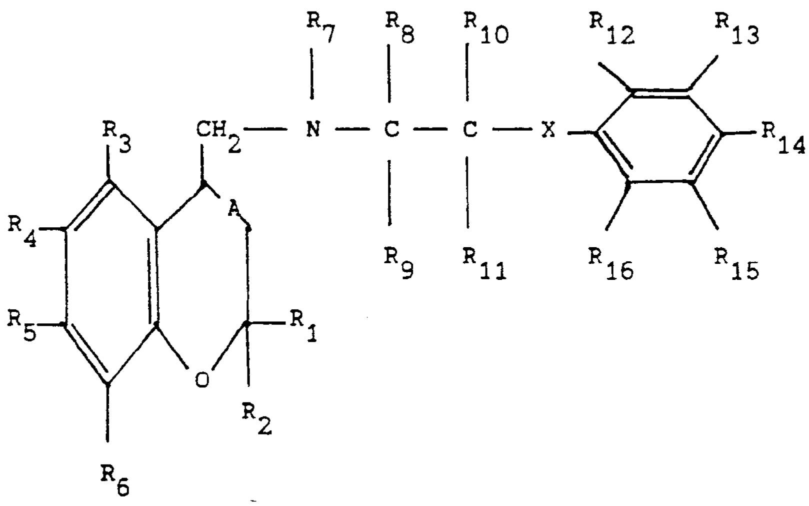

dans laquelle

- A représente notamment une liaison simple,
- $R_1$, $R_2$, $R_8$, $R_9$, $R_{10}$ et $R_{11}$ peuvent représenter des atomes d'hydrogène,
- $R_3$, $R_4$, $R_8$ et $R_8$ peuvent être des atomes d'hydrogène ou des radicaux alcoyloxy,
- $R_{12}$ à $R_{16}$ peuvent être entre autres des atomes d'hydrogène, des radicaux alcoyloxy ou 2 de ces radicaux adjacents peuvent former un radical méthylènedioxy,
- et $-NR_7-CR_8R_9-CR_{10}R_{11}-X-$ peut représenter un radical pipérazinyle.

Il a été trouvé que les produits de formule générale (I) dans laquelle

- $R_1$ représente un atome d'hydrogène ou d'halogène ou un radical hydroxy, alcoyloxy, nitro, amino ou alcoylsulfonamido, bis(alcoylsulfonyl)amino, ou acylamino,
- X représente un atome d'azote ou un radical $>$CH-
- R représente un radical de formule générale :

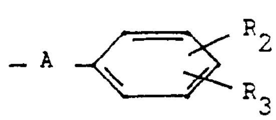

(II)

dans laquelle

A représente une liaison simple, un radical méthylène ou, lorsque X est un atome d'azote A peut représenter un radical carbonyle, et $R_2$ et $R_3$ identiques ou différents représentent un atome d'hydrogène ou d'halogène, ou un radical hydroxy, alcoyle, alcoyloxy, nitro, amino, alcoylsulfonamido, bis(alcoylsulfonyl-)amino, acylamino, sulfamoyle, ou cyano, ou forment ensemble lorsqu'ils sont adjacents, un radical méthylènedioxy ou éthylènedioxy, ou bien R représente un radical pyridyle ou représente un radical 2H-benzimidazolone-2 yle si X représente >CH-,

– R′ et R″ identiques représentent des atomes d'hydrogène ou des radicaux alcoyle,

ainsi que leurs sels, entraînent une augmentation des périodes réfractaires particulièrement intéressante qui correspond aux effets antifibrillants des produits antiarythmiques de la classe III selon la classification de VAUGHAN WILLIAMS.

Dans la formule générale (I), lorsque $R_1$ et, $R_2$ et $R_3$ (dans le symbole R), représentent un atome d'halogène, celui-ci peut être choisi parmi le fluor, le chlore, le brome ou l'iode; lorsque $R_1$, $R_2$ ou $R_3$ représentent ou contiennent des radicaux alcoyle ou acyle, ces derniers peuvent être droits ou ramifiés et contiennent 1 à 4 atomes de carbone.

Il est entendu que les produits de formule générale (I) présentent des formes isomères, et que ces isomères et leurs mélanges entrent dans le cadre de la présente invention.

Selon l'invention, les produits de formule générale (I) peuvent être obtenus par action d'un produit de formule générale :

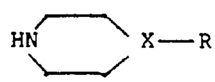

(III)

ou de son sel, dans laquelle X et R sont définis comme précédemment, sur un dérivé de benzopyranne de formule générale :

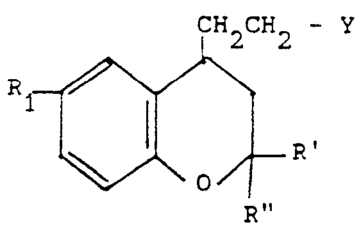

(IV)

dans laquelle $R_1$, R′ et R″ sont définis comme précédemment et Y représente un atome d'halogène ou un radical alcoylsulfonyloxy ou arylsulfonyloxy.

On opère avantageusement en présence d'un agent accepteur d'acide. Il est également possible d'opérer sans accepteur d'acide, en présence de 2 équivalents du produit de formule générale (III).

Lorsque Y représente un atome d'halogène, il peut être choisi parmi les atomes de chlore ou de brome.

Lorsque Y représente un radical alcoylsulfonyloxy, il représente notamment le radical méthylsulfonyloxy et lorsqu'il représente un radical arylsulfonyloxy, il peut être entre autres le radical p.toluènesulfonyloxy.

A titre d'accepteur d'acide, on utilise avantageusement un hydroxyde de métal alcalin ou alcalinoterreux (soude ou potasse par exemple), un carbonate de métal alcalin (bicarbonate de sodium, carbonate de potassium par exemple), ou une base organique azotée telle que la triéthylamine par exemple.

La réaction s'effectue dans un solvant inerte tel qu'une cétone (acétone, butanone par exemple), un éther (tétrahydrofuranne ou dioxanne par exemple), un alcool (méthanol ou éthanol par exemple), un hydrocarbure (hexane ou toluène par exemple), l'acétonitrile, le diméthylformamide ou le diméthylsulfoxyde, ou dans un mélange de tels solvants, à une température comprise entre 20°C et la température de reflux du mélange réactionnel.

Il est entendu que dans les cas où $R_1$, $R_2$ et/ou $R_3$ (dans R) représentent un radical amino, ce dernier est préalablement protégé. De même lorsque $R_2$ et/ou $R_3$ représentent un radical hydroxy, il est préférable de protéger ce radical préalablement à la réaction.

La protection s'effectue par tout groupement compatible et dont la mise en oeuvre et l'élimination n'altèrent pas le reste de la molécule. Notamment on opère selon les méthodes décrites par T.W. Greene, Protective Groups in Organic Synthesis, A. Wiley - Interscience Publication (1981), ou par Mc Omie, Protective Groups in Organic Chemistry, Plenum Press (1973).

Selon l'invention, les produits de formule générale (I) pour lesquels les radicaux $R_1$, $R_2$ et/ou $R_3$ représen-

tent un radical hydroxy peuvent également être obtenus à partir du produit de formule générale (I) correspondant pour lequel le radical $R_1$, $R_2$ et/ou $R_3$ à transformer représente un radical alcoyloxy, par traitement en milieu acide concentré.

La réaction s'effectue généralement par traitement par l'acide bromhydrique, ou un mélange d'acides, par exemple par traitement par un mélange acide bromhydique - acide acétique, à la température de reflux du mélange réactionnel.

Selon l'invention, les produits de formule générale (I) pour lesquels les symboles $R_1$, $R_2$ et/ou $R_3$ représentent un radical amino, alcoylsulfonamido, bis(alcoylsulfonyl)amino, ou acylamino peuvent également être obtenus par hydrogénation catalytique en milieu acide du dérivé du benzopyranne de formule générale (I) pour lequel le radical $R_1$, $R_2$ et/ou $R_3$ à transformer représente un radical nitro, puis lorsque l'on veut obtenir un produit de formule générale (I) pour lequel $R_1$, $R_2$ et/ou $R_3$ représentent un radical alcoylsulfonamido, bis(alcoylsulfonyl)amino ou acylamino, on transforme le dérivé aminé obtenu respectivement par sulfonylation ou par acylation.

L'hydrogénation s'effectue avantageusement à une température comprise entre 20 et 50°C, dans un acide comme par exemple l'acide acétique ou l'acide chlorhydrique, dans un solvant organique tel qu'un alcool (méthanol, éthanol, isopropanol par exemple) dans un mélange de solvants, ou en milieu hydroorganique (alcool - eau par exemple). Il est également possible d'opérer directement dans l'acide, sans addition supplémentaire d'un solvant.

A titre de catalyseur, on utilise généralement le palladium, l'oxyde de platine ou le nickel de Raney.

Eventuellement on opère sous pression.

La sulfonylation ou l'acylation s'effectuent respectivement par action d'une forme activée d'un acide $alkSO_3H$ ou alk'COOH (alk et alk' étant des radicaux alcoyle), notamment l'halogénure d'acide (chlorure d'acide par exemple) ou l'anhydride, et l'on opère en présence d'un accepteur d'acide tel qu'une base organique azotée comme une trialcoylamine (triéthylamine par exemple) ou comme la pyridine, dans un solvant organique inerte tel qu'un solvant chloré (dichlorométhane, chloroforme par exemple), un éther (éther éthylique, tétrahydrofuranne par exemple) ou dans un mélange de ces solvants, à une température comprise entre -70 et +40°C.

Eventuellement on opère sous azote.

Lorsque l'on veut obtenir le produit de formule générale (I) pour lequel $R_1$, $R_2$ et/ou $R_3$ représentent un radical bis(alcoylsulfonyl)amino, on opère en présence de 2 équivalents du dérivé de l'acide sulfonique correspondant.

Selon l'invention, les produits de formule générale (I) pour lesquels A représente un radical carbonyle, peuvent aussi être préparés par action d'un acide benzoïque de formule générale :

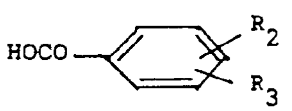

(V)

dans laquelle $R_2$ et $R_3$ sont définis comme précédemment, ou d'un dérivé réactif de cet acide, sur un dérivé du benzopyranne de formule générale :

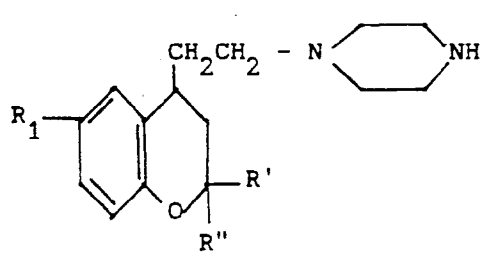

(VI)

dans laquelle $R_1$, R' et R" sont définis comme précédemment.

Il est entendu que, lorsque $R_1$, $R_2$ et/ou $R_3$ représentent des radicaux amino ou hydroxy, ceux-ci sont protégés préalablement à la réaction.

La protection et l'élimination des radicaux protecteurs s'effectue dans les conditions décrites précédemment pour le procédé qui consiste à faire réagir les produits de formule générale (III) et (IV).

Lorsque l'on met en oeuvre l'acide de formule générale (V), on opère en présence d'un agent de condensation peptidique tel qu'un carbodiimide (dicyclohexylcarbodiimide par exemple) ou le N,N'carbonyldiimidazole, ou l'éthoxy-2 éthoxycarbonyl-1 dihydro-1,2 quinoléine, dans un solvant organique tel qu'un éther

(tétrahydrofuranne), un amide (diméthylformamide), un nitrile (acétonitrile) ou un solvant chloré (par exemple le dichlorométhane), à une température comprise entre -10 et +20°C.

Lorsque l'on met en oeuvre un dérivé réactif de l'acide de formule générale (V), il est possible de faire réagir l'anhydride, un anhydride mixte, un halogénure d'acide ou un ester activé. On opère alors soit en milieu organique, éventuellement en présence d'un accepteur d'acide tel qu'une base organique azotée (par exemple une trialcoylamine ou une pyridine) dans un solvant tel que cité ci-dessus, ou un mélange de ces solvants à une température comprise entre 0 et +20°C, soit en milieu hydroorganique en présence d'un agent alcalin de condensation tel qu'un carbonate ou un bicarbonate de métal alcalin ou alcalino terreux, à une température comprise entre 5 et 20°C.

Les produits de formule générale (III) peuvent être préparés selon les méthodes décrites par :

- V. NACCI et coll., Farmaco Ed. Sci., 328(5), 399 (1973),
- P.C. JAIN et coll., J. Med. Chem., 10, 813 (1967),
- J. CRAIG et coll., Org. Synth., 5, 88 (1973),
- demande de brevet japonais JA 82 093 962
- demande de brevet néerlandais NE 65 10 107
- brevet américain US 4 421 753
- J.A. Kiritsy et coll., J. Med. Chem., 21 (12), 1301 (1978)
- D. Kohlbach Arhiv. Hem. Farm. 11, 99 (1937)

décrites ci-après dans les exemples, ou par analogie avec ces méthodes.

Les produits de formule générale (IV) peuvent être obtenus par action d'un agent d'halogénation ou d'une forme activée d'un acide alcoylsulfonique ou arylsulfonique sur un dérivé de l'hydroxyalcoyl-4 benzopyranne de formule générale :

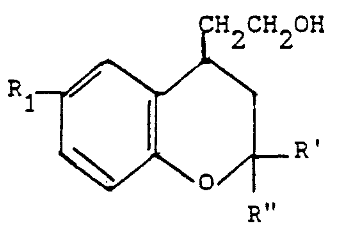

(VII)

dans laquelle $R_1$, R′ et R″ sont définis comme précédemment.

Lorsque l'on veut préparer un produit de formule générale (IV) pour lequel Y est un atome d'halogène, les agents d'halogénation peuvent être choisis parmi le chlorure de thionyle ou les dérivés halogénés du phosphore, tels que l'oxychlorure de phosphore ou le tribromure de phosphore. Il est également possible de faire réagir le bromure d'allyle en présence de NN′-carbonyldiimidazole.

Lorsque l'on veut préparer un produit de formule générale (IV) dans laquelle Y est alcoylsulfonyloxy ou arylsulfonyloxy, on fait avantageusement réagir l'anhydride ou l'halogénure de l'acide correspondant.

La réaction s'effectue généralement en présence d'une base organique azotée telle que la triéthylamine ou la pyridine, dans un solvant organique tel qu'un solvant chloré (chlorure de méthylène par exemple), un éther (tétrahydrofuranne, dioxanne par exemple), en opérant à une température comprise entre 0°C et la température de reflux du mélange réactionnel.

Les produits de formule générale (IV) dans laquelle $R_1$ est un radical nitro peuvent être obtenus par nitration d'un dérivé de formule générale (IV) pour lequel $R_1$ est un atome d'hydrogène.

On opère avantageusement au moyen du mélange acide nitrique - acide acétique à une température comprise entre 0 et 20°C.

Les produits de formule générale (IV) dans laquelle $R_1$ est un radical hydroxy peuvent également être obtenus à partir d'un produit de formule générale (IV) dans laquelle $R_1$ est un radical alcoyloxy, par traitement en milieu acide concentré. On opère dans les conditions décrites précédemment pour l'obtention d'un produit de formule générale (I) pour lequel $R_1$ représente un radical hydroxy à partir du produit correspondant pour lequel $R_1$ est un radical alcoyloxy.

Le dérivé de l'hydroxyalcoyl-4 benzopyranne de formule générale (VII) peut être préparé par réduction de l'ester correspondant de formule générale :

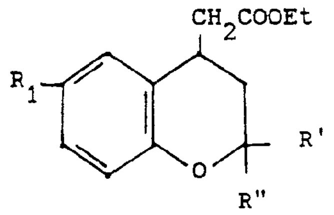

(VIII)

dans laquelle $R_1$, R'et R" sont définis comme précédemment.

On opère généralement au moyen d'hydrure d'aluminium et de lithium dans un solvant organique tel qu'un éther (tétrahydrofuranne par exemple) à une température comprise entre 0 et 30°C.

L'ester de formule générale (VIII) peut être obtenu par réduction du dérivé du benzopyranne de formule générale :

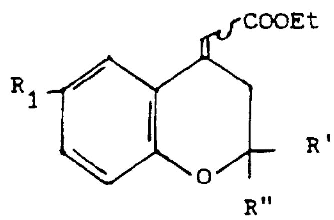

(IX)

dans laquelle $R_1$, R' et R" sont définis comme précédemment.

On opère par hydrogénation catalytique en présence de palladium, dans un solvant organique tel qu'un alcool (méthanol, éthanol par exemple), à une température comprise entre 10 et 50°C.

Le dérivé du benzopyranne de formulé générale (IX) peut être préparé par réaction de WITTIG, à partir d'un dérivé de la chromannone-4 de formule générale :

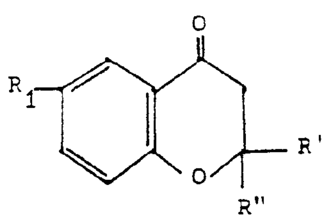

(X)

dans laquelle $R_1$, R' et R" sont définis comme précédemment.

On opère avantageusement au moyen de diéthylphosphonoacétate d'éthyle en présence d'hydrure de sodium, dans un solvant organique tel qu'un éther (tétrahydrofuranne ou diméthoxyéthane par exemple ) à une température comprise entre 0°C et la température de reflux du mélange réactionnel.

Le dérivé de la chromannone-4 de formule générale (X) dans laquelle $R_1$ est autre que l'hydrogène, peut être préparé par application de la méthode décrite par PFEIFFER et coll., Chem. Ber., 58 (1954), ou selon les méthodes décrites par G.P. Ellis, Heterocyclic compounds, chromenes, chromanones and chromones, John Wiley and Sons (1977).

Le dérivé de la chromannone-4 de formule générale (X) dans laquelle $R_1$ est un atome de fluor, peut être préparé selon la méthode décrite dans la demande de brevet français 2 588 860.

Les dérivés de la chromannone-4 de formule générale (X) dans laquelle $R_1$ est un radical amino, alcoylsulfonamido, bis(alcoylsulfonyl)amino, trifluorométhylsulfonamido ou acylamino, peuvent être obtenus à partir du dérivé de la chromannone-4 de formule générale (X) pour lequel $R_1$ est un radical nitro, par analogie avec les méthodes décrites pour la préparation des produits de formule générale (I) pour lesquels le radical $R_1$ est défini comme ci-dessus.

La diméthyl-2,2 chromannone-4 peut être obtenue selon la méthode décrite dans le brevet belge 844 943.

Les produits de formule générale (V) peuvent être préparés selon, ou par analogie, avec les méthodes décrites dans :

- J. Am. Chem. Soc., 70, 4177 (1948),
- EP 023 578

Les dérivés du benzopyranne de formule générale (VI) peuvent être obtenus par action de la pipérazine sur un dérivé du benzopyranne de formule générale (IV).

La réaction s'effectue dans les conditions décrites précédemment pour la réaction des produits de formule générale (III) avec les dérivés du benzopyranne de formule générale (IV), en présence d'un excès de pipérazine (2 équivalents), sans addition supplémentaire d'un accepteur d'acide.

Les énantiomères des produits selon l'invention peuvent être séparés selon les méthodes connues.

On opère notamment par préparation de l'énantiomère du dérivé de l'hydroxyéthylbenzopyranne de formule générale (VII) qui est transformé en produit de formule générale (I) selon le procédé décrit précédemment.

Le dérivé optiquement actif de formule générale (VII) est obtenu par préparation d'un amide optiquement actif de formule générale :

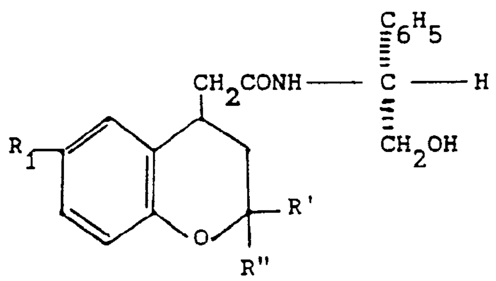

(XI)

dans laquelle $R_1$, R′ et R″ sont définis comme précédemment, séparation des isomères par chromatographie, hydrolyse de l'isomère recherché puis réduction de l'acide obtenu.

L'hydrolyse de l'isomère du produit de formule générale (XI) peut être effectuée par toute méthode connue qui n'altère pas le reste de la molécule, on opère avantageusement en milieu acide (acide acétique, acide chlorhydrique en mélanges) à la température de reflux du mélange réactionnel.

La réduction de l'acide en alcool est mise en oeuvre selon les méthodes habituelles. Notamment on utilise le diboranne à titre d'agent réducteur et l'on opère avantageusement dans un éther tel que le tétrahydrofuranne à des températures comprises entre 0 et 30°C.

Le produit de formule générale (XI) peut être préparé à partir de l'acide de formule générale :

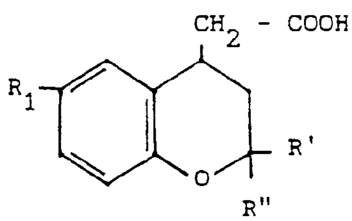

(XII)

dans laquelle $R_1$, R′ et R″ sont définis comme précédemment, par toute méthode connue pour préparer un amide à partir d'un acide.

On opère avantageusement au moyen du chlorure de l'acide de formule générale (XII) (qui peut être préparé in situ) dans un solvant organique inerte tel qu'un solvant chloré (dichlorométhane par exemple) en présence d'un agent accepteur d'acide comme une base organique azotée (triéthylamine par exemple), à une température comprise entre 0 et 30°C.

L'acide de formule générale (XII) peut être obtenu à partir de l'ester correspondant, par toute méthode connue pour obtenir un acide à partir d'un ester sans toucher au reste de la molécule.

On effectue notamment la saponification de l'ester de formule générale (VIII) par la potasse, dans le méthanol à la température de reflux du mélange réactionnel.

Le chlorure d'acide est préparé par traitement de l'aci- de correspondant par le chlorure de thionyle à la température de reflux du mélange réactionnel.

Les nouveaux dérivés du benzopyranne selon l'invention peuvent être purifiés le cas échéant, par des méthodes physiques telles que la cristallisation ou la chromatographie.

Les produits selon l'invention peuvent être transformés en sels d'addition avec les acides. Le sel formé précipite après concentration éventuelle de sa solution, il est séparé par filtration, décantation ou lyophilisation. Selon le procédé de la pré- sente invention, les produits sont généralement obtenus à l'état de chlorhydrate. Ces sels peuvent être libérés et transformés en sels d'autres acides selon les méthodes habituelles.

Comme exemples de sels pharmaceutiquement acceptables peuvent être cités les sels d'addition avec des acides minéraux (chlorhydrates, bromhydrates, sulfates, nitrates, phosphates) ou organiques (succinates, fumarates, acétates, propionates, maléates, méthanesulfonates, p.toluènesulfonates, iséthionates) ou des dérivés de substitution de ces composés.

Les produits selon l'invention manifestent des propriétés antiarythmiques et antifibrillantes particulièrement intéressantes, caractéristiques de la classe III de VAUGHAN WILLIAMS, se traduisant par un allongement des périodes réfractaires.

Ils provoquent notamment, in vitro sur muscle papillaire de cobaye, une augmentation comprise entre 5% et des valeurs supérieures à 50 %, de la durée du potentiel d'action initial, selon la technique des mesures d'enregistrement du potentiel d'action intracellulaire décrite par E. CORABOEUF et S. WEIDMANN, C.R. Soc. Biol., 143, 1329 (1949).

Par ailleurs, les dérivés du benzopyranne selon l'invention manifestent une faible toxicité. Ils se sont généralement montrés atoxiques à 300 mg/kg par voie orale chez la souris.

Plus spécialement intéressants sont les produits de formule générale (I) pour lesquels :

– $R_1$ représente un atome d'hydrogène, de chlore ou de fluor, ou un radical hydroxy, méthoxy, nitro, amino ou méthylsulfonylamino,

– X représente un atome d'azote ou un radical >CH-,

– R représente un radical de formule générale (II) dans laquelle:
A est une liaison simple ou un radical méthylène, ou lorsque X est un atome d'azote, A peut aussi représenter un radical carbonyle et, $R_2$ et $R_3$ identiques ou différents sont situés en position 3 et/ou 4 et représentent un atome d'hydrogène ou de fluor, ou un radical hydroxy, méthyle, méthoxy, nitro, amino, méthylsulfonamido, bis(méthylsulfonyl)amino, acétylamino, sulfamoyle ou cyano, ou forment ensemble lorsqu'ils sont adjacents un radical méthylènedioxy ou éthylènedioxy, ou bien

R représente un radical pyridyle ou représente un radical 2H-benzimidazolone-2-yle si X représente >CH-,

– R′ et R″ sont identiques et représentent des atomes d'hydrogène ou des radicaux méthyle sous leurs formes isomères ou leurs mélanges et notamment les produits suivants :

– La [(dihydro-3,4 2H-benzopyran-1 yl-4)-2 éthyl]-1 (diméthoxy-3,4 phényl)-4 pipéridine, ses formes isomères et leurs mélanges.

– L′ [(amino-6 dihydro-3,4 2H-benzopyran-1 yl-4)-2 éthyl]-1 (diméthoxy-3,4 phényl)-4 pipéridine, ses formes isomères et leurs mélanges.

– La [(dihydro-3,4 2H-benzopyran-1 yl-4)-2 éthyl]-1 méthylsulfonamido-4 phényl)-4 pipéridine, ses formes isomères et leurs mélanges.

– La [(dihydro-3,4 2H-benzopyran-1 yl-4)-2 éthyl]-1 (méthylsulfonamido-4 phényl)-4 pipérazine, ses formes isomères et leurs mélanges.

– La {[(dihydro-3,4 2H-benzopyran-1 yl-4)-2 éthyl]-1 pipéridinyl-4}-1 dihydro-1,3 2H-benzimidazolone-2, ses formes isomères et leurs mélanges.

Les exemples suivants illustrent la présente invention.

Dans les exemples qui suivent, sauf mention spéciale, les chromatographies sont mises en oeuvre sur gel de silice (60 - 200μ).

## EXEMPLE 1

On chauffe à reflux pendant 3 heures, 9,7 g de [bromo-2 éthyl]-4 dihydro-3,4 2H-benzopyranne, 11,12 g de dichlorhydrate de (diméthoxy-3,4 phényl)-1 pipérazine, 15,6 g de carbonate de potassium sec et 6,8 g d'iodure de potassium dans 300 $cm^3$ de butanone-2.

On filtre le mélange réactionnel sur verre fritté puis on évapore le solvant sous pression réduite (5,2 KPa). On extrait l'huile obtenue par 300 cm3 de dichlorométhane puis on lave par 40 cm3 d'une solution 1N de soude, lave à l'eau puis sèche la phase organique sur sulfate de magnésium.

Après évaporation, on reprend l'huile obtenue par 100 $cm^3$ d'éthanol et ajoute 35 $cm^3$ d'une solution 2N d'acide chlorhydrique dans l'éthanol. Aprés filtration du précipité formé, sur verre fritté, on obtient 14 g de dichlorhydrate de [(dihydro-3,4 2H-benzopyran-1 yl-4)-2 éthyl]-1 (diméthoxy-3,4 phényl)-4 pipérazine sous forme d'un solide blanc fondant avec décomposition aux environs de 226°C.

La (diméthoxy-3,4 phényl)-1 pipérazine peut être préparée selon la technique décrite par P.C. JAIN et coll.,

J. Med. Chem., 10, 813 (1967).

Le bromo-2 éthyl-4 dihydro-3,4 2H-benzopyranne peut être préparé de la manière suivante :

A 115 $cm^3$ d'acétonitrile on ajoute, sous agitation, 13,8 g de (dihydro-3,4 2H-benzopyran-1 yl-4)-2 éthanol, puis 91,2 g de bromure d'allyle et enfin 12,6 g de NN'-carbonyldiimidazole.

On agite 3 heures 10 minutes à 20°C environ puis 2 heures à reflux.

Le mélange réactionnel est ensuite concentré sous pression réduite (5,2 KPa) et le résidu obtenu est chromatographié sur une colonne de 5,5 cm de diamètre contenant 200 g de gel de silice en éluant par 550 $cm^3$ de dichlorométhane et en recueillant des fractions de 100 $cm^3$. Les fractions comprises entre 350 et 550 $cm^3$ sont concentrées à sec.

On obtient ainsi 17,7 g de (bromo-2 éthyl)-4 dihydro-3,4 2H-benzopyranne sous forme d'une huile brun clair.

Spectre de RMN du proton (250 MHz, $CDCl_3$, δ en ppm) :

6,8 à 7,2 (mt, 4H aromatiques)
4,21 (mt, -O-C$\underline{H}_2$-)
3,55 (mt, -C$\underline{H}_2$-Br

3,08 (mt, >CH-)
1,92 et 2,92 (mt, -$CH_2$- en -3)
2,08 et 2,34 (mt, -C$\underline{H}_2$$CH_2$Br)

Le (dihydro-3,4 2H-benzopyran-1 yl-4)-2 éthanol peut être préparé de la manière suivante :

A 5,96 g d'hydrure de lithium et d'aluminium, on ajoute 500 $cm^3$ de tétrahydrofuranne et refroidit à 0°C. On ajoute alors, sous agitation, 17,25 g de (dihydro-3,4 2H-benzopyran-1 yl-4)-2 éthanoate d'éthyle dans 60 $cm^3$ de tétrahydrofuranne.

Après 1 heure d'agitation à 20°C, on hydrolyse sous agitation par addition de sulfate de sodium hydraté (10 $H_2O$) jusqu'à précipitation puis on laisse reposer le mélange réactionnel pendant 15 heures.

Après filtration du précipité formé et évaporation du solvant sous pression réduite, on isole 13,8 g de (dihydro-3,4 2H-benzopyran-1 yl-4)-2 éthanol sous forme d'une huile brune.

Spectre de RMN (250 MHz, $CDCl_3$, δ en ppm) :

6,8 à 7,2 (mt, 4H aromatiques)
4,22 (mt, -O-C$\underline{H}_2$-)
3,83 (mt, -C$\underline{H}_2$-OH)

3,04 (mt, >CH-)
1,83 et 2,90 (mt, -$CH_2$- en -3 et -C$\underline{H}_2$-$CH_2$OH)
1,62 (s, -O$\underline{H}$)

Le (dihydro-3,4 2H-benzopyran-1 yl-4) éthanoate d'éthyle peut être préparé de la manière suivante :

50,6 g (dihydro-3,4 2H-benzopyran-1 ylidène-4) acétate d'éthyle (E,Z) dans 1 litre de méthanol, sont hydrogénés à 20°C sous pression atmosphérique, en présence de 5,06 g de palladium sur charbon (10 %).

Après filtration sur KIESELGUHR et concentration à sec sous pression réduite (5,2 KPa), on obtient 48,8 g de (dihydro-3,4 2H-benzopyran-1 yl-4) éthanoate d'éthyle sous forme d'une huile jaune pâle.

Spectre de RMN (250 MHz, $CDCl_3$, δ en ppm) :

6,75 à 7,2 (mt, 4H aromatiques)
4,98 (q + mt, -O-C$\underline{H}_2$- + -CO-OC$\underline{H}_2$-$CH_3$)

3,37 (mt, >C$\underline{H}$-)
2,53 et 2,82 (dd, -C$\underline{H}_2$-CO-)
1,87 et 2,18 (mt, -$CH_2$- en -3)
1,30 (t, -COO-$CH_2$-C$\underline{H}_3$)

Le (dihydro-3,4 2H-benzopyran-1 ylidène-4) acétate d'éthyle (E,Z) peut être préparé de la manière suivante :

A 1 litre de tétrahydrofuranne anhydre, on ajoute, sous agitation, 20,4 g d'hydrure de sodium (80 %) puis par petites portions 153 g de diéthylphosphonoacétate d'éthyle tout en maintenant la température du mélange réactionnel aux environs de 20°C.

Puis la solution jaune claire ainsi obtenue est additionnée de 45 g de chromannone-4 dans 100 $cm^3$ de tétrahydrofuranne anhydre en maintenant la température en dessous de 0°C. Après 22 heures à 20°C, on concentre sous pression réduite le mélange réactionnel puis extrait l'huile obtenue par 2 fois 700 $cm^3$ de dichlorométhane. La phase organique est lavée à l'eau puis séchée sur sulfate de magnésium et concentrée à sec sous pression réduite. Le résidu d'évaporation est chromatographié sur une colonne de 9 cm de diamètre, contenant 1,6 kg de gel de silice, en éluant par 6,3 litres d'un mélange cyclohexane-acétate d'éthyle (90-10 en volume) et en

recueillant des fractions de 250 $cm^3$. Les fractions comprises entre 2,8 l et 6,3 l sont concentrées à sec.

On obtient ainsi 50,6 g d'un mélange d'isomères E et Z du (dihydro-3,4 2H-benzopyran-1 ylidène-4) acétate d'éthyle sous forme d'une huile jaune pâle.

Spectre de RMN (400 MHz, $CDCl_3$, δ en ppm) :

Isomère E (75 %) :

6,8 à 7,61 (mt, 4H aromatiques)
6,36 (s, =C<u>H</u>-CO-)
4,23 (mt, -O-C$\underline{H_2}$-)
4,23 (mt, -CO-OC$\underline{H_2}$-$CH_3$)
3,41 (mt, -$CH_2$- en -3)
1,32 (mt, -CO-$OCH_2$-C$\underline{H_3}$)

Isomère Z (25 %) :

6,8 à 7,83 (mt, 4H aromatiques)
5,61 (s, =C<u>H</u>-CO-)
4,38 (t, -O-C$\underline{H_2}$-)
4,23 (mt, -CO-OC$\underline{H_2}$-$CH_3$)
2,65 (t, -$CH_2$- en -3)
1,32 (mt, -CO-$OCH_2$-C$\underline{H_3}$)

<u>EXEMPLE 2</u>

L'isomère A du dichlorhydrate de (dihydro-3,4 2H-benzopyran-1 yl-4)-2 éthyl]-1 (diméthoxy-3,4 phényl)-4 pipérazine est préparé en opérant comme à l'exemple 1 mais à partir de 0,86 g de l'isomère A du (bromo-2 éthyl)-4 dihydro-3,4 2H-benzopyranne, de 1,10 g de dichlorhydrate de (diméthoxy-3,4 phényl)-1 pipérazine puis de 1,38 g de carbonate de potassium sec et de 0,6 g d'iodure de potassium dans 15 $cm^3$ de butanone-2.

On reprend l'huile obtenue par 9 $cm^3$ d'éthanol et ajoute 1,5 $cm^3$ d'une solution 5N d'acide chlorhydrique dans l'isopropanol. Le précipité ainsi obtenu est filtré puis cristallisé dans 150 $cm^3$ de méthanol.

On obtient ainsi 1,28 g l'isomère A du dichlorhydrate de [(dihydro-3,4 2H-benzopyran-1 yl-4)-2 éthyl]-1 (diméthoxy-3,4 phényl)-4 pipérazine sous forme d'un solide blanc fondant avec décomposition aux environs de 219-221°C.

$[\alpha]_D^{20}$ = -13° ± 0,8 (c = 0,736, $H_2O$).

L'isomère A du (bromo-2 éthyl)-4 dihydro-3,4 2H-benzopyranne peut être préparé en opérant comme à l'exemple 1 mais à partir de 0,96 g de l'isomère A du (dihydro -3,4 2H-benzopyran-1 yl-4)-2 éthanol, de 4,9 g de bromure d'allyle et de 0,875 g de NN'-carbonyldiimidazole dans 8 $cm^3$ d'acétonitrile. Le résidu obtenu est chromatographié sur une colonne de 2 cm de diamètre contenant 25 g de gel de silice en éluant par 120 $cm^3$ de dichlorométhane et en recueillant des fractions de 30 $cm^3$. Les fractions comprises entre 60 et 120 $cm^3$ sont concentrées à sec.

On obtient ainsi 0,86 g de l'isomère A du (bromo-2 éthyl)-4 dihydro-3,4 2H-benzopyranne sous forme d'une huile incolore qui est utilisée telle quelle dans l'étape suivante.

L'isomère A du (dihydro-3,4 2H-benzopyran-1 yl-4)-2 éthanol peut être préparé de la manière suivante :

A 1,26 g de l'isomère A de l'acide (dihydro-3,4 2H-benzopyran-1 yl-4) éthanoïque, on ajoute 5 $cm^3$ de tétrahydrofuranne anhydre et refroidit à 0°C. On ajoute alors, lentement et sous agitation, 17,5 $cm^3$ d'une solution 1M de diboranne dans le tétrahydrofuranne. Après la fin de l'introduction, on laisse remonter à 20°C la température du mélange réactionnel et maintient ainsi sous agitation pendant 4 heures 30, puis on ajoute 10 $cm^3$ de méthanol au mélange réactionnel. L'huile obtenue après élimination du solvant est chromatographiée sur une colonne de 2 cm de diamètre contenant 60 g de gel de silice en utilisant comme éluant 210 $cm^3$ d'un mélange dichlorométhane-acétone (80-20 en volume) et en recueillant des fractions de 30 $cm^3$. Les fractions comprises entre 150 et 210 $cm^3$ sont concentrées à sec.

On obtient ainsi 1,07 g de l'isomère A du (dihydro-3,4 2H-benzopyran-1 yl-4)-2 éthanol sous forme d'une huile incolore qui est utilisée telle quelle dans l'étape suivante.

L'isomère A de l'acide (dihydro-3,4 2H-benzopyran-1 yl-4) éthanoïque peut être préparé de la manière suivante :

A 3 g de l'isomère A de N-(hydroxy-2 phényl-1 éthyl)-(dihydro-3,4 2H-benzopyran-1 yl-4) éthanamide dans 10 $cm^3$ d'acide acétique pur, on ajoute 10 $cm^3$ d'acide chlorhydrique concentré et on chauffe à reflux pendant 1 heure 30. On concentre à sec le mélange réactionnel sous pression réduite (5,2 KPa) puis on extrait à l'éther éthylique. La phase éthérée lavée à l'eau est concentrée à sec sous pression réduite et le résidu obtenu est repris par 110 $cm^3$ d'une solution 1N de soude puis extrait par 200 $cm^3$ de dichlorométhane.

La liqueur alcaline est acidifiée par 12 $cm^3$ d'acide chlorhydrique concentré, puis extraite par le dichlorométhane. La phase organique est ensuite lavée à l'eau puis séchée sur sulfate de magnésium. Le résidu obtenu après concentration à sec est cristallisé dans un mélange d'acétate d'isopropyle et d'éther de pétrole (40-60°) et donne 1,95 g de l'isomère A de l'acide (dihydro-3,4 2H-benzopyran-1 yl-4) éthanoïque sous forme de cristaux crème fondant à 77-78°C.

$[\alpha]_D^{20}$ = -18,5° ± 0,5 (c = 1,136, éthanol)

L'isomère A de N-(hydroxy-2 phényl-1 éthyl)-(dihydro-3,4 2H-benzopyran-1 yl-4) éthanamide peut être préparé en opérant de la manière suivante :

A 3,45 g de (R)-(-)amino-2 phényl-2 éthanol dans 25 $cm^3$ de dichlorométhane, on ajoute 3,9 $cm^3$ de triéthylamine et refroidit à 5°C puis on introduit goutte à goutte une solution de 5,3 g du chlorure de l'acide (dihydro-3,4 2H-benzopyran-1 yl-4) éthanoïque dans 10 $cm^3$ de dichlorométhane. On maintient la température du mélange réactionnel à 0°C pendant 4 heures puis laisse reposer, sous agitation, pendant 15 heures à 20°C. Après lavage de la phase organique par une solution 1N d'acide chlorhydrique et ensuite par une solution 1N de soude, la solution chlorométhylénique est lavée à l'eau puis séchée sur sulfate de magnésium.

Après filtration et concentration à sec sous pression réduite (5,2 KPa), on obtient un résidu constitué d'un mélange de diastéréoisomères qui est ensuite chromatographié sur une colonne de 9 cm de diamètre contenant 1 kg de gel de silice (32-63 μ) en utilisant comme éluant 8,4 litres d'un mélange dichlorométhane-éthanol (95-5 en volumes) et en recueillant des fractions de 125 $cm^3$. Les fractions comprises entre 4 et 5 litres concentrées à sec donnent 3,2 g de l'isomère A de N-(hydroxy-2 phényl-1 éthyl)-(dihydro-3,4 2H-benzopyran-1 yl-4) éthanamide sous forme de cristaux blancs fondant à 143°C.

$[\alpha]_D^{20}$ = -43,9° ± 0,5 (c = 1,504, éthanol)

Le chlorure de l'acide (dihydro-3,4 2H-benzopyran-1 yl-4) éthanoïque peut être préparé de la manière suivante :

On chauffe au reflux pendant 6 heures, 5 g de l'acide (dihydro-3,4 2H-benzopyran-1 yl-4) éthanoïque dans 12 $cm^3$ de chlorure de thionyle.

Après distillation, on obtient 4,1 g de l'acide (dihydro-3,4 2H-benzopyran-1 yl-4) éthanoïque sous forme d'une huile jaune dont le point d'ébullition est de 110-120°C sous 2,63 Pa.

L'acide (dihydro-3,4 2H-benzopyran-1 yl-4) éthanoïque peut être préparé de la manière suivante :

On chauffe au reflux pendant 2 heures 15, 15 g de (dihydro-3,4 2H-benzopyran-1 yl-4) éthanoate d'éthyle et 23,5 g de potasse en pastilles dans 250 $cm^3$ de méthanol.

Le mélange réactionnel est concentré sous pression ré- duite (5,2 KPa), repris à l'eau puis extrait par 300 $cm^3$ d'éther éthylique. La phase aqueuse est ensuite acidifiée par 45 $cm^3$ d'une solution d'acide chlorhydrique concentré puis on extrait par 600 $cm^3$ de dichlorométhane. La phase organique est ensuite lavée à l'eau puis séchée sur sulfate de magnésium. Le résidu obtenu après concentration à sec est recristallisé dans un mélange (50-50 en volume) d'acétate d'iso propyle et d'éther de pétrole (40-60°) et donne 9 g d'acide (dihydro-3,4 2H-benzopyran-1 yl-4) éthanoïque sous forme de cristaux blancs fondant à 90°C.

## EXEMPLE 3

L'isomère B du dichlorhydrate de [(dihydro-3,4 2H-benzopyran-1 yl-4)-2 éthyl]-1 (diméthoxy-3,4 phényl)-4 pipérazine peut être préparé en opérant comme à l'exemple 2 mais à partir de 0,72 g de l'isomère B du [(bromo-2 éthyl)-1]-4 dihydro-3,4 2H-benzopyranne, de 0,92 g de dichlorhydrate de (diméthoxy-3,4 phényl)-1 pipérazine puis de 1,38 g de carbonate de potassium sec et de 0,51 g d'iodure de potassium dans 15 cm3 de butanone-2. On obtient 0,80 g de l'isomère B du dichlorhydrate de [(dihydro-3,4 2H-benzopyran-1 yl-4)-2 éthyl]-1 (diméthoxy-3,4 phényl)-4 pipérazine sous forme d'un solide blanc fondant avec décomposition aux environs de 219-220°.

$[\alpha]_D^{20}$ = + 12,4 ±0,6° (c = 0,840, eau).

L'isomère B du [(bromo-2 éthyl)-1]-4-dihydro-3,4 2H-benzopyranne peut être préparé en opérant comme à l'exemple 2 mais à partir de 0,76 g de l'isomère B du (dihydro-3,4 2H-benzopyran-1 yl-4)-2 éthanol, de 3,88 g de bromure d'allyle et de 0,69 g de N,N'-carbonyldiimidazole dans 6,4 cm3 d'acétonitrile. Après purification comme décrit à l'exemple 2, on obtient 0,72 g de l'isomère B du [(bromo-2 éthyl)-1]-4 dihydro-3,4 2H-benzopyranne sous forme d'une huile jaune pâle.

L'isomère B du (dihydro-3,4 2H-benzopyran-1 yl-4)-2 éthanol peut être préparé en opérant comme à l'exemple 2 mais à partir de 0,90 g de l'isomère B de l'acide (dihydro-3,4 2H-benzopyran-1 yl-4) éthanoïque dans 4 cm3 de tétrahydrofuranne et de 12,6 cm3 d'une solution 1M de diboranne dans le tétrahydrofuranne. On obtient ainsi 0,76 g de (dihydro-3,4 2H-benzopyran-1 yl-4)-2 éthanol sous forme d'une huile incolore.

L'isomère B de l'acide (dihydro-3,4 2H-benzopyran-1 yl-4) éthanoïque peut être préparé en opérant comme

à l'exemple 2 mais à partir de 3 g de l'isomère B de N-(hydroxy-1 phényl-2 éthyl)-2 (dihydro-3,4 2H-benzopyran-1 yl-4) éthanamide dans 10 cm3 d'acide acétique pur contenant 10 cm3 d'acide chlorhydrique concentré. On obtient ainsi 0,96 g de l'isomère B de l'acide (dihydro-3,4 2H-benzopyran-1 yl-44) éthanoïque sous forme de cristaux crème fondant à 77-78°C.

$[\alpha]_D^{20}$ = + 17,4 ±0,5° (c = 1,046, éthanol).

L'isomère B de N-(hydroxy-1 phényl-2 éthyl)-2 dihydro-3,4 2H-benzopyran-1 yl-4) éthanamide peut être préparé en opérant comme à l'exemple 2 mais en recueillant les fractions de chromatographie comprises entre 6 litres et 8,4 litres. Après concentration à sec sous pression réduite (5,2 kPa), on obtient 3,05 g de N-(hydroxy-1 phényl-2 éthyl)-2 dihydro-3,4 2H-benzopyran-1 yl-4) éthanamide sous forme de cristaux blancs fondant à 140°C.

$[\alpha]_D^{20}$ = 6,5 ±0,3° (c = 1,498, éthanol).

EXEMPLE 4

On opère comme à l'exemple 1 à partir de 1,5 g de bromo-2 éthyl)-4 dihydro-3,4 2H-benzopyranne, de 1,03 g de phényl-1 pipérazine puis de 0,8 g de carbonate de potassium sec et de 0,1 g d'iodure de potassium dans 50 cm³ de butanone-2.

On reprend l'huile par 30 cm³ d'éthanol et ajoute 5 cm³ d'une solution 2N d'acide chlorhydrique dans l'éthanol.

On obtient ainsi 1,4 g de dichlorhydrate de [(dihydro-3,4 2H-benzopyran-1 yl-4)-2 éthyl]-1 phényl-4 pipérazine sous forme d'un solide blanc fondant à 206°C.

EXEMPLE 5

On opère comme à l'exemple 1 à partir de 1 g de (bromo-2 éthyl)-4 dihydro-3,4 2H-benzopyranne, de 0,63 g de phényl-4 pipéridine, de 0,54 g de carbonate de potassium sec puis de 0,7 g d'iodure de potassium dans 50 cm³ de butanone-2.

On reprend l'huile obtenue par 10 cm³ d'éthanol et ajoute 1,8 cm³ d'une solution 2N d'acide chlorhydrique dans l'éthanol.

Après addition de 50 cm³ d'éther éthylique puis filtration du précipité formé, on obtient 1,05 g de chlorhydrate de [(dihydro-3,4 2H-benzopyran-1 yl-4)-2 éthyl]-1 phényl pipéridine sous forme d'un solide blanc fondant à 250°C.

EXEMPLE 6

On opère comme à l'exemple 1, à partir de 3,35 g de (bromo-2 éthyl)-4 dihydro-3,4 2H-benzopyranne, de 3 g de chlorhydrate de (fluoro-4 phényl)-4 pipéridine et de 3,8 g de carbonate de potassium sec puis de 2,31 g d'iodure de potassium dans 115 cm³ de butanone-2.

Le résidu obtenu est purifié par chromatographie sur une colonne de 5,5 cm de diamètre contenant 300 g de gel de silice en utilisant un mélange de dichlorométhane et d'acétone (75-25 en volumes) comme éluant et en recueillant des fractions de 250 cm³. Les fractions comprises entre 1000 et 3000 cm³ sont concentrées à sec. L'huile obtenue est reprise par 16 cm³ d'éthanol puis additionnée de 3 cm³ d'une solution 5N d'acide chlorhydrique dans l'isopropanol.

On concentre à sec et recristallise dans 10 cm³ de butanone-2. On obtient ainsi 2,05 g de chlorhydrate de [(dihydro-3,4 2H-benzopyran-1 yl-4)-2 éthyl]-1 (fluoro-4 phényl)-4 pipérazine sous forme de cristaux blancs fondantà environ 190°C avec décomposition.

Le chlorhydrate de (fluoro-4 phényl)-4 pipéridine peut être préparé selon la méthode décrite dans la demande de brevet NE 65 10 107.

EXEMPLE 7

On opère comme à l'exemple 1 mais à partir de 2 g de (bromo-2 éthyl)-4 dihydro-3,4 2H-benzopyranne, de 1,96 g de chlorhydrate de (méthoxy-3 phényl)-1 pipérazine et de 2,28 g de carbonate de potassium sec puis de 1,3 g d'iodure de potassium dans 70 cm3 de butanone-2. Le résidu obtenu est filtré sur une colonne de 4,4 cm de diamètre contenant 100 g de gel de silice en éluant avec 450 cm3 d'acétate d'éthyle et en recueillant des fractions de 30 cm3. Les fractions comprises entre 210 et 450 cm3 sont concentrées à sec. On reprend l'huile obtenue par 40 cm3 d'éthanol et ajoute 3,1 cm3 d'une solution 5N d'acide chlorhydrique dans l'isopropanol.

On obtient ainsi 2,4 g de dichlorhydrate de [(dihydro-3,4 2H-benzopyran-1 yl-4)-2 éthyl]-1 (méthoxy-3 phényl)-4 pipérazine sous forme d'un solide fondant à 190°C.

EXEMPLE 8

On opère comme à l'exemple 1, à partir de 7 g de (bromo-2 éthyl)-4 dihydro-3,4 2H-benzopyranne, de 7,08 g de chlorhydrate de (méthoxy-4 phényl)-1 pipérazine et de 6 g de carbonate de potassium sec puis de 4,81 g d'iodure de potassium dans 230 cm3 de butanone-2. La base obtenue est purifiée par chromatographie sur une colonne de 6 cm de diamètre contenant 300 g de gel de silice, en éluant par un mélange de dichlorométhane et d'acétone (80-20 en volumes) et en recueillant des fractions de 250 cm3. Les fractions comprises entre 750 cm3 et 2500 cm3 sont concentrées à sec. L'huile obtenue est reprise par 400 cm3 d'un mélange d'acétone et d'éthanol (60-40 en volumes) et additionnée de 24,7 cm3 d'une solution aqueuse 2N d'acide chlorhydrique. Le précipité formé est recristallisé dans 200 cm3 d'éthanol. On obtient ainsi 8,3 g de dichlorhydrate de [dihydro-3,4 2H-benzopyran-1 yl)-4 éthyl]-1 (méthoxy-4 phényl)-4 pipérazine sous forme d'un solide blanc dont le point de fusion est de 175°C.

EXEMPLE 9

On opère comme à l'exemple 1 mais à partir de 5 g de (bromo-2 éthyl)-4 dihydro-3,4 2H-benzopyranne, de 4,3 g de (nitro-4 phényl)-1 pipérazine et de 1,43 g de carbonate de potassium sec puis de 3,43 g d'iodure de potassium dans 200 cm3 de butanone-2. Le résidu obtenu est chromatographié sur une colonne de 4,4 cm de diamètre contenant 100 g de gel de silice en éluant avec un mélange de dichlorométhane et d'acétate d'éthyle (50-50 en volumes) et en recueillant des fractions de 20 cm3. Les fractions comprises entre 100 et 300 cm3 sont concentrées à sec. Le résidu obtenu est cristallisé dans 30 cm3 d'acétate d'isopropyle. On obtient ainsi 3,48 g de [(dihydro-3,4 2H-benzopyran-1 yl-4)-2 éthyl]-1 (nitro-4 phényl)-4 pipérazine sous forme d'un solide jaune fondant à 110°C.

EXEMPLE 10

On opère comme à l'exemple 1, mais à partir de 5,85 g de (bromo-2 éthyl)-4 dihydro-3,4 2H-benzopyranne, de 5,9 g de (nitro-4 phényl)-4 pipéridine et de 6,6 g de carbonate de potassium sec puis de 4 g d'iodure de potassium dans 200 cm3 de butanone-2.

Le résidu obtenu est purifié par chromatographie sur une colonne de 4,4 cm de diamètre contenant 100 g de gel de silice en utilisant un mélange de dichlorométhane et d'acétone (50-50 en volumes) comme éluant et en recueillant des fractions de 20 cm3. Les fractions comprises entre 200 et 380 cm3 sont concentrées à sec.

Après cristallisation dans 35 cm3 d'acétate d'isopropyle, on obtient 4,25 g de [(dihydro-3,4 2H-benzopyran-1 yl-4)-2 éthyl]-1 (nitro-4 phényl)-4 pipéridine sous forme d'un solide blanc fondant à 100°C.

EXEMPLE 11

On porte à reflux, pendant 7 heures 30 minutes, 5 g de (bromo-2 éthyl)-4 dihydro-3,4 2H-benzopyranne, de 6,4 g de bromhydrate de (pipérazin-1 yl)-4 benzènesulfonamide, de 5,53 g de carbonate de potassium sec et 3,32 g d'iodure de potassium dans 100 cm3 de butanone-2.

L'insoluble est filtré et lavé deux fois avec 50 cm3 de butanone-2, puis est repris dans 200 cm3 d'eau distillée et 3,4 cm3 d'une solution à 35% d'acide chlorhydrique concentré de façon à obtenir un pH de 7. On filtre à nouveau et lave l'insoluble avec 50 cm3 puis deux fois 20 cm3 d'éthanol. On obtient 6,7 g d'une poudre grise qui est recristallisée dans 120 cm3 de méthoxy-2 éthanol bouillant. Après 2 heures de repos à 20°C on filtre les cristaux et lave avec deux fois 20 cm3 d'éthanol, deux fois avec 20 cm3 d'acétone puis deux fois 20 cm3 d'éther éthylique pour obtenir 3,54 g d'une poudre blanche qui est recristallisée dans 70 cm3 de méthoxy-2 éthanol. Après repos pendant 1 heure 30 minutes à 20°C, les cristaux sont essorés et lavés successivement avec deux fois 10 cm3 de méthoxy-2 éthanol, deux fois 10 cm3 d'éthanol, deux fois 10 cm3 d'acétone et enfin deux fois 10 cm3 d'éther éthylique. On obtient ainsi 2,47 g de {[dihydro-3,4 2H-benzopyran-1 yl-4)-2 éthyl-1]-1 pipérazinyl-4}-4 benzènesulfonamide sous forme d'un solide blanc fondant à 225°C.

Le bromydrate de (pipérazin-1 yl)-4 benzène sulfonamide peut être préparé selon la méthode de D. Kohlbach., Arhiv. Hem. Farm., 11, 99 (1937).

EXEMPLE 12

On opère comme à l'exemple 1, à partir de 15 g de (bromo-2 éthyl)-4 dihydro-3,4 2H-benzopyranne, de 11,24 g de (cyano-4 phényl)-1 pipérazine, de 8,3 g de carbonate de potassium sec puis de 9,95 g d'iodure de potassium dans 200 cm3 de butanone-2. On chauffe au reflux pendant 7 heures. L'huile obtenue est reprise dnas 100 cm3 d'eau distillée, extraite trois fois avec 100 cm3 de dichlorométhane puis la phase organique est séchée sur du sulfate de magnésium. L'évaporation du dichlorométhane permet d'obtenir 24 g d'une huile. Cette huile est reprise dans 100 cm3 d'un mélange d'acétate d'isopropyle et d'éther isopropylique (50-50 en volumes). Après repos 16 heures à 10°C, on élimine 0,3 g d'un solide. Le filtrat est dilué avec 50 cm3 d'éther isopropylique. Après repos (1 heure à 10°C), on obtient 17,9 g d'un solide crème. Ce solide est recristallisé dans 100 cm3 d'un mélange d'acétate d'isopropyle et d'éther isopropylique (60-40 en volumes). Après repos (1 heure à 20°C), on obtient 1,55 g de (cyano-4 phényl)-1 [(dihydro-3,4 2H-benzopyran-1 yl-4)-2 éthyl]-4 pipérazine sous forme d'un solide blanc fondant à 106°C.

La (cyano-4 phényl)-1 pipérazine peut être préparée selon la méthode de J.A. Kiritsy, D.K. Yung, J. Med. Chem., 21 (12), 1301 (1978).

EXEMPLE 13

On opère comme à l'exemple 1 à partir de 2 g de (bromo-2 éthyl)-4 dihydro-3,4 2H-benzopyranne, de 1,84 g de (diméthoxy-3,5 phényl)-1 pipérazine et de 0,56 g de carbonate de potassium sec puis de 1,4 g d'iodure de potassium dans 70 cm3 de butanone-2. Le résidu obtenu est chromatographié sur une colonne de 4,4 cm de diamètre contenant 100 g de gel de silice en utilisant comme éluant un mélange de dichlorométhane et d'acétate d'éthyle (50-50 en volumes) et en recueillant des fractions de 20 cm3. Les fractions comprises entre 200 et 400 cm3 sont concentrées à sec. On reprend l'huile obtenue par 30 cm3 d'éthanol et ajoute 1,8 cm3 d'une solution 5N d'acide chlorhydrique dans l'isopropanol. On filtre les cristaux formés et on obtient ainsi 1,9 g de dichlorhydrate de [(dihydro-3,4 2H benzopyran-1 yl-4)-2 éthyl]-1 (diméthoxy-3,5 phényl)-4 pipérazine sous forme d'un solide fondant à 208°C.

EXEMPLE 14

On opère comme à l'exemple 1 à partir de 0,72 g de (bromo-2 éthyl)-4 méthoxy-6 dihydro-3,4 2H-benzopyranne, de 0,79 g de dichlorhydrate de (diméthoxy-3,4 phényl)-1 pipérazine et de 1,1 g de carbonate de potassium sec puis de 0,05 g d'iodure de potassium dans 30 $cm^3$ de butanone-2.

On reprend l'huile obtenue par 20 $cm^3$ d'éthanol et ajoute 2,5 $cm^3$ d'une solution 2N d'acide chlorhydrique dans l'éthanol.

On obtient 0,8 g de dichlorhydrate de [(méthoxy-6 dihydro-3,4 2H-benzopyran-1 yl-4)-2 éthyl]-1 (diméthoxy-3,4 phényl)-4 pipérazine sous forme d'un solide blanc fondant à 185°C.

Le (bromo-2 éthyl)-4 méthoxy-6 dihydro-3,4 2H-benzopyranne peut être préparé en opérant comme à l'exemple 1 mais à partir de 0,72 g de (méthoxy-6 dihydro-3,4 2H-benzopyran-1 yl-4)-2 éthanol, de 0,56 g de NN'-carbonyldiimidazole et de 3 $cm^3$ de bromure d'allyle dans 20 $cm^3$ d'acétonitrile. L'huile ainsi obtenue est chromatographiée sur une colonne de 4,4 cm de diamètre contenant 60 g de gel de silice en éluant par 120 $cm^3$ de dichlorométhane et en recueillant des fractions de 30 $cm^3$. Les fractions comprises entre 90 et 120 $cm^3$ sont concentrées à sec.

On obtient ainsi 0,72 g de (bromo-2 éthyl)-4 méthoxy-6 dihydro-3,4 2H-benzopyranne sous forme d'une huile jaune pâle.

Spectre de RMN (90 MHz, $CDCl_3$, δ en ppm) :

6,6 à 6,8 (mt, 3H aromatiques)
4,15 (mt, $-O-C\underline{H}_2-$)
3,75 (s, $-O-C\underline{H}_3$)
3,55 (mt, $-C\underline{H}_2-Br$)
3,05 (mt, $>CH-$)
1,5 et 2,6 (mt, $-CH_2-$ en -3 et $-C\underline{H}_2CH_2Br$)

Le (méthoxy-6 dihydro-3,4 2H-benzopyran-1 yl-4)-2 éthanol peut être préparé en opérant comme à l'exemple 1 mais à partir de 0,29 g d'hydrure de lithium et d'aluminium, de 0,94 g de (méthoxy-6 dihydro-3,4 2H-benzopyran-1 yl-4) éthanoate d'éthyle dans 50 $cm^3$ de tétrahydrofuranne. On obtient ainsi 0,72 g de (méthoxy-6 dihydro-3,4 2H-benzopyran-1 yl-4)-2 éthanol sous forme d'une huile incolore.

Spectre de RMN (90 MHz, $CDCl_3$, δ en ppm) :

6,6 à 6,8 (mt, 3H aromatiques)
4,15 (mt, $-O-C\underline{H}_2-$)
3,85 (mt, $-C\underline{H}_2-OH$)
3,75 (s, $-O-C\underline{H}_3$)
2,85 - 3,10 (mt, $\diagdown$ CH-)
1,55 - 2,3 (mt, $-CH_2-$ en -3 et $-C\underline{H}_2CH_2OH$)
1,45 (s, $-O-\underline{H}$)

Le (méthoxy-6 dihydro-3,4 2H-benzopyran-1 yl-4) éthanoate d'éthyle peut être préparé en opérant comme à l'exemple 1 mais à partir de 1 g d'un mélange de méthoxy-6 dihydro-3,4 2H-benzopyran-1 ylidène-4 acétate d'éthyle (E,Z), de 0,2 g de palladium sur charbon (10 %) dans 100 $cm^3$ de méthanol.

On obtient ainsi 0,96 g de (méthoxy-6 dihydro-3,4 2H-benzopyran-1 yl-4) éthanoate d'éthyle sous forme d'une huile jaune pâle.

Spectre de RMN (250 MHz, $CDCl_3$, $\delta$ en ppm) :
6,65 à 6,8 (mt, 4H aromatiques)
4,26 et 4,20 ( mt + q, $-O-C\underline{H}_2-$ + $-CO-OC\underline{H}_2-CH_3$)
3,76 (s, $-O-C\underline{H}_3$)
3,36 (mt, $\diagdown$CH-)
2,53 et 2,81 (dd, $-C\underline{H}_2-CO_2-$)
2,13 et 1,83 (mt, $-C\underline{H}_2-$ en 3)
1,3 (t, $-CH_2-C\underline{H}_3$).

Le méthoxy-6 dihydro-3,4 2H-benzopyran-1 ylidène-4 acé- tate d'éthyle (E,Z) peut être préparé en opérant comme à l'exemple 1 mais à partir de 18,87 g de diéthylphosphonoacétate d'éthyle, de 2,52 g d'hydrure de sodium et de 5 g de méthoxy-6 chromannone-4 [préparé selon PFEIFFER et coll. Chem Ber,58 (1954)] dans 190 $cm^3$ de tétrahydrofuranne.

L'huile obtenue est chromatographiée sur une colonne de 6,5 cm de diamètre contenant 500 g de gel de silice en utilisant comme éluant 3 litres d'un mélange cyclohexane-acétate d'éthyle (90-10 en volume) et en recueillant des fractions de 250 $cm^3$. Les fractions comprises entre 1,9 litre et 2,4 litres sont concentrées à sec.

On obtient ainsi 5,52 g d'un mélange d'isomères E et Z du méthoxy-6 dihydro-3,4 2H-benzopyran-1 ylidène-4 acétate d'éthyle sous forme d'une huile jaune pâle.

EXEMPLE 15

On opère comme à l'exemple 1 mais à partir de 1,5 g de (bromo-2 éthyl)-4 dihydro-3,4 2H-benzopyranne, de 1,5 g de (diméthoxy-3,4 phényl)-4 pipéridine puis de 1,61 g de carbonate de potassium sec et de 1 g d'iodure de potassium dans 50 $cm^3$ de butanone-2.

On reprend l'huile obtenue par 15 $cm^3$ d'éthanol et ajoute 2,7 $cm^3$ d'une solution 2N d'acide chlorhydrique dans l'éthanol.

On obtient ainsi 1,6 g de chlorhydrate de [(dihydro-3,4 2H-benzopyran-1 yl-4)-2 éthyl]-1 (diméthoxy-3,4 phényl)-4 pipéridine sous forme d'un solide blanc fondant à 237°C.

La (diméthoxy-3,4 phényl)-4 pipéridine peut être préparée selon la méthode décrite par V. NACCI et coll., Farmaco Ed. Sci., 328(5), 399-410 (1973).

EXEMPLE 16

On opère comme à l'exemple 2 mais à partir de 1,5 g de l'isomère A du (bromo-2 éthyl)-4 dihydro-3,4 2H-benzopyranne, de 1,78 g de chlorhydrate de (diméthoxy-3,4 phényl)-4 pipéridine puis de 1,71 g de carbonate de potassium sec et de 1,03 g d'iodure de potassium dans 50 cm3 de butanone-2. L'huile obtenue est reprise par 50 cm3 d'acétate d'éthyle. La phase organique est lavée par 10 cm3 d'une solution 1N de soude puis à l'eau et ensuite séchée sur sulfate de magnésium. On concentre à sec sous pression réduite et le résidu est chromatographié sur une colonne de 3 cm de diamètre, contenant 25 g de gel de silice, en éluant par un mélange de dichlorométhane et d'acétone (70-30 en volumes) et en recueillant des fractions de 100 cm3. Les cinq premières fractions sont concentrées à sec. On dissout à chaud l'huile obtenue dans le minimum d'alcool isopropylique puis cette solution est ajoutée à une solution chaude de 0,696 g d'acide fumarique dans l'alcool isopropylique. Après retour à une température voisine de 20°C, on filtre les cristaux formés puis recristallise dans 100 cm3 d'isopropanol. On obtient ainsi 2,4 g d'isomère A du fumarate de [(dihydro-3,4 2H-benzopyran-1 yl-4)-2 éthyl]-1 (diméthoxy-3,4 phényl)-4 pipéridine sous forme d'un solide blanc fondant à 170°C.

$[\alpha]_D^{20}$ = -12,3 ±0,9° (c = 0,509, eau).

L'isomère A du (bromo-2 éthyl)-4 dihydro-3,4 2H-benzopyranne peut être préparé en opérant comme décrit à l'exemple 2.

EXEMPLE 17

L'isomère B du fumarate de [(dihydro-3,4 2H-benzopyran-1 yl-4)-2 éthyl]-1 (diméthoxy-3,4 phényl)-4 pipéridine est préparé comme à l'exemple 16 mais à partir de 1,5 g de l'isomère B du (bromo-2 éthyl)-4 dihydro-3,4 2H-benzopyranne, de 1,78 g de dichlorhydrate de (diméthoxy-3,4 phényl)-4 pipéridine puis de 1,71 g de carbonate de potassium et de 1,03 g d'iodure de potassium dans 50 cm3 de butanone-2.

On obtient ainsi 2,4 g d'isomère B du fumarate de [(dihydro-3,4 2H-benzopyran-1 yl-4)-2 éthyl]-1 (diméthoxy-3,4 phényl)-4 pipéridine sous forme d'un solide blanc fondant à 170°C.

$[\alpha]_D^{20}$ = +11,9 ± 1,1° (c = 0,42, eau).

EXEMPLE 18

On opère comme à l'exemple 1, à partir de 2 g de (bromo-2 éthyl)-4 fluoro-6 dihydro-3,4 2H-benzopyranne, de 1,99 g de chlorhydrate de diméthoxy-3,4 phényl)-4 pipéridine et de 1,06 g de carbonate de potassium sec puis de 1,27 g d'iodure de potassium dans 70 $cm^3$ de butanone-2.

Le résidu obtenu est purifié par chromatographie sur une colonne de 4,4 cm de diamètre contenant 100 g de gel de silice en utilisant un mélange de dichlorométhane et d'acétone (50-50 en volumes) comme éluant et en recueillant des fractions de 20 $cm^3$. Les fractions comprises entre 190 et 410 $cm^3$ sont concentrées à sec. L'huile obtenue est reprise par 30 $cm^3$ d'éthanol puis additionnée de 1,46 $cm^3$ d'une solution 5,5N d'acide chlorhydrique dans l'isopropanol. Le produit obtenu (2,44 g) est recristallisé dans 30 cm3 d'éthanol et on obtient ainsi 1,7 g de chlorhydrate de [(fluoro-6 dihydro-3,4 2H-benzopyran-1 yl-4)-2 éthyl]-1 (diméthoxy-3,4 phényl)-4 pipéridine sous forme d'un solide blanc fondant à 200°C.

Le (bromo-2 éthyl)-4 fluoro-6 dihydro-3,4 2H-benzopyranne peut être préparé en opérant comme à l'exemple 1 mais à partir de 6,32 g de (fluoro-6 dihydro-3,4 2H-benzopyran-1 yl-4)-2 éthanol, de 5,22 g de NN'-carbonyldiimidazole et de 19,2 $cm^3$ de bromure d'allyle dans 50 $cm^3$ d'acétonitrile. L'huile ainsi obtenue est chromatographiée sur une colonne de 4,4 cm de diamètre contenant 100 g de gel de silice en éluant par 240 $cm^3$ de dichlorométhane et en recueillant des fractions de 30 $cm^3$. Les fractions comprises entre 150 et 240 $cm^3$ sont concentrées à sec.

On obtient ainsi 6,3 g de (bromo-2 éthyl)-4 fluoro-6 dihydro-3,4 2H-benzopyranne sous forme d'une huile jaune pâle.

Spectre de RMN (250 MHz, $CDCl_3$, δ en ppm) :

6,7 à 6,95 (mt, 3H aromatiques)
4,17 (mt, -O-C$\underline{H}_2$-)
3,54 (mt, -C$\underline{H}_2$-Br)
3,08 (mt, >CH-)
2,33 et 2,06 (mt, -C$\underline{H}_2$-$CH_2$Br)
2,15 et 1,77 (mt, -C$\underline{H}_2$- en 3)

Le (fluoro-6 dihydro-3,4 2H-benzopyran-1 yl-4)-2 éthanol peut être préparé en opérant comme à l'exemple 1 mais à partir de 2,55 g d'hydrure de lithium et d'aluminium, de 8 g de (fluoro-6 dihydro-3,4 2H-benzopyran-1 yl-4) éthanoate d'éthyle dans 180 $cm^3$ de tétrahydrofuranne. On obtient ainsi 6,59 g de (fluoro-6 dihydro-3,4 2H-benzopyran-1 yl-4)-2 éthanol sous forme d'une huile incolore.

Spectre de RMN (200 MHz, $CDCl_3$, δ en ppm) :

6,7 à 6,9 (mt, 3H aromatiques)
4,14 (mt, -O-C$\underline{H}_2$-)
3,85 (mt, -C$\underline{H}_2$-OH)
3 (mt, >CH-)
1,7 - 2,2 (mt, -$CH_2$- en -3 et -C$\underline{H}_2$$CH_2$OH)

Le (fluoro-6 dihydro-3,4 2H-benzopyran-1 yl-4) éthanoate d'éthyle peut être préparé en opérant comme à l'exemple 1 mais à partir de 14,9 g d'un mélange de fluoro-6 dihydro-3,4 2H-benzopyran-1 ylidène-4 acétate d'éthyle (E,Z et endo), et de 0,750 g de palladium sur charbon (10 %) dans 400 $cm^3$ de méthanol.

On obtient ainsi 14,6 g de (fluoro-6 dihydro-3,4 2H-benzopyran-1 yl-4) éthanoate d'éthyle sous forme d'une huile jaune pâle.

Spectre de RMN (250 MHz, $CDCl_3$, δ en ppm) :

6,75 à 6,90 (mt, 3H aromatiques)

4,13 et 4,18 (mt + q, -O-C$\underline{H}_2$- + -CO-OC$\underline{H}_2$-$CH_3$)

3,32 (mt, > CH-)

2,51 et 2,75 (mt, > C$\underline{H}$-)

7,80 et 2,11 (mt, -C$\underline{H}_2$- en 3)

1,27 (t, -CO-O-$CH_2$-C$\underline{H}_3$)

Le fluoro-6 dihydro-3,4 2H-benzopyran-1 ylidène-4 acétate d'éthyle (E,Z et endo) peut être préparé en opérant comme à l'exemple 1 mais à partir de 44,1 g de diéthylphosphonoacétate d'éthyle, de 5,37 g d'hydrure de sodium 80% et de 11,9 g de fluoro-6 chromannone-4 [préparé selon la demande de brevet FR 2 588 860] dans 300 $cm^3$ de tétrahydrofuranne.

L'huile obtenue est chromatographiée sur une colonne de 4,4 cm contenant 100 g de gel de silice en utilisant comme éluant 0,58 litre d'un mélange cyclohexane-acétate d'éthyle (90-10 en volumes) et en recueillant des fractions de 20 cm3. Les fractions comprises entre 100 et 580 cm3 sont concentrées à sec.

On obtient ainsi 15,9 g d'un mélange d'isomères E, Z et endo du fluoro-6 dihydro-3,4 2H-benzopyran-1 ylidène-4 acétate d'éthyle sous forme d'une huile jaune pâle.

Spectre de RMN (200 MHz, $CDCl_3$, $\delta$ en ppm).

Pics caractéristiques pour chacun des produits :

| | | |
|---|---|---|
| Isomère E (65%) | ) | 3,35 (dt, -C$\underline{H}_2$- en 3) |
| | ) | 6,26 (s large, -CH=) |
| Isomère Z (15%) | ) | 2,61 (dt, -C$\underline{H}_2$- en 3) |
| | ) | 5,73 (s large, -CH=) |
| Isomère endo (20%) | ) | 3,35 (s, -C$\underline{H}_2$-$CO_2$-) |
| | ) | 4,76 (d, -O-C$\underline{H}_2$-) |
| | ) | 5,8 (mt, -CH=) |

EXEMPLE 19

On opère comme à l'exemple 1 à partir de 2 g de [(bromo-2 éthyl)-4 chloro-6 dihydro-3,4 2H-benzopyranne, de 1,87 g de chlorhydrate de (diméthoxy-3,4 phényl)-4 pipéridine et de 1,07 g de carbonate de potassium sec puis de 1,21 g d'iodure de potassium dans 60 cm3 de butanone-2. Le résidu obtenu est filtré sur une colonne de 4,4 cm de diamètre contenant 100 g de gel de silice en éluant avec un mélange de dichlorométhane et d'acétone (50-50 en volumes) et en recueillant des fractions de 30 cm3. Les fractions comprises entre 270 et 540 cm3 sont concentrées à sec. On reprend l'huile obtenue par 30 cm3 d'éthanol et ajoute 1,3 cm3 d'une solution 5,5N d'acide chlorhydrique dans l'isopropanol puis 200 cm3 d'éther éthylique.

On obtient ainsi 2,74 g de chlorhydrate de [(chloro-6 dihydro-3,4 2H-benzopyran-1 yl-4)-4 éthyl]-1 (diméthoxy-3,4 phényl)-4 pipéridine sous forme d'un solide fondant à 191°C.

Le (bromo-2 éthyl)-4 chloro-6 dihydro-3,4 2H-benzopyranne peut être préparé en opérant comme à l'exemple 1 mais à partir de 5,5 g de (chloro-6 dihydro-3,4 2H-benzopyran-1 yl-4)-2 éthanol, de 4,2 g de NN'-carbonyldiimidazole et de 1,55 $cm^3$ de bromure d'allyle dans 30 $cm^3$ d'acétonitrile. L'huile ainsi obtenue est chromatographiée sur une colonne de 4,4 cm de diamètre contenant 100 g de gel de silice en éluant par un mélange de dichlorométhane et de cyclohexane (30-70) et en recueillant des fractions de 30 $cm^3$. Les fractions comprises entre 180 et 300 $cm^3$ sont concentrées à sec.

On obtient 5,67 g d'un résidu qui est à nouveau repurifié par chromatographie en phase liquide sur une colonne de 4 cm de diamètre et de 30 cm de hauteur contenant de la silice 40$\mu$ en utilisant comme phase mobile un mélange de dichlorométhane et de cyclohexane (20-80 en volumes) et un débit de phase mobile de 70 cm3/mn. Les fractions comprises entre 910 cm3 et 1330 cm3 sont concentrées à sec.

On obtient ainsi 3,8 g de (bromo-2 éthyl)-4 chloro-6 dihydro-3,4 2H-benzopyranne sous forme d'une huile jaune.

Spectre de RMN (250 MHz, $CDCl_3$, $\delta$ en ppm) :

7,13 (d, 1H, aromatique en 5)

7,07 (dd, aromatique en 7)

6,76 (d, 1H, aromatique en 8)
4,19 (mt, 2H, $-O-C\underline{H}_2-$)
3,55 (mt, 2H, $-C\underline{H}_2-Br$)
3,08 (mt, 1H, $>C\underline{H}-$)
2,34 et 2,06 (mt, $-C\underline{H}_2-CH_2Br$)
2,14 et 1,78 (mt, $-C\underline{H}_2-$ en 3)

Le (chloro-6 dihydro-3,4 2H-benzopyran-1 yl-4)-2 éthanol peut être préparé en opérant comme à l'exemple 1 mais à partir de 1,97 g d'hydrure de lithium et d'aluminium, de 6,6 g de (chloro-6 dihydro-3,4 2H-benzopyran-1 yl-4) éthanoate d'éthyle dans 120 $cm^3$ de tétrahydrofuranne. On obtient ainsi 5,5 g de (méthoxy-6 dihydro-3,4 2H-benzopyran-1 yl-4)-2 éthanol sous forme d'une huile incolore.

Spectre de RMN (250 MHz, $CDCl_3$, $\delta$ en ppm) :
6,75 (d, 1H, aromatique en 8)
7,06 (dd, 1H, aromatique en 7)
7,15 (d, 1H, aromatique en 5)
4,20 (mt, $-O-C\underline{H}_2-$)
3,82 (t, $-C\underline{H}_2-OH$)
3,02 (mt, $>C\underline{H}-$)
1,85 et 2,13 (mt, $-C\underline{H}_2-$ en -3 et $-C\underline{H}_2CH_2OH$)
1,78 et 2,05 (mt, $-C\underline{H}_2CH_2OH$)
1,55 (s, $-O-\underline{H}$)

Le (chloro-6 dihydro-3,4 2H-benzopyran-1 yl-4) éthanoate d'éthyle peut être préparé en opérant comme à l'exemple 1 mais à partir de 6,83 g d'un mélange de chloro-6 dihydro-3,4 2H-benzopyran-1 ylidène-4 acétate d'éthyle (E) et de 0,7 g de palladium sur charbon (10 %) dans 200 $cm^3$ d'acétate d'éthyle.

On obtient ainsi 6,6 g de (méthoxy-6 dihydro-3,4 2H-benzopyran-1 yl-4) éthanoate d'éthyle sous forme d'une huile jaune pâle.

Spectre de RMN (400 MHz, $CDCl_3$, $\delta$ en ppm) :
7,3 (d, 1H, aromatique en 5)
7,23 (dd, 1H, aromatique en 7)
6,92 (d, 1H, aromatique en 8)
4,35 (mt, $-C\underline{H}_2-O-$ et $-CO_2C\underline{H}_2-$)
3,5 (mt, $>C\underline{H}-$)
2,7 et 2,95 (dd, $-C\underline{H}_2-CO_2-$)
2,02 et 2,3 (mt, $-C\underline{H}_2-$ en 3)
1,47 (t, $-C\underline{H}_3$)

Le (chloro-6 dihydro-3,4 2H-benzopyran-1 ylidène-4 acétate d'éthyle (E) peut être préparé comme décrit à l'exemple 1, mais à partir de 4,11 g d'hydrure de sodium (80%), de 33,7 g de diéthylphosphonoacétate d'éthyle et de 10 g de chloro-6 chromannone-4 dans 250 cm3 de tétrahydrofuranne. Le résidu obtenu est chromatographié sur une colonne de 4,4 cm de diamètre, contenant 200 g de gel de silice, la phase mobile étant un mélange de cyclohexane et d'acétate d'éthyle (90-10 en volumes). Les fractions comprises entre 400 cm3 et 1120 cm3 sont concentrées à sec.

On obtient ainsi 10 g de (chloro-6 dihydro-3,4 2H-benzopyran-1 ylidène-4) acétate d'éthyle isomère E (contenant 3% d'isomère Z) sous forme d'une huile jaune pâle.

Spectre de RMN (250 MHz, $CDCl_3$, $\delta$ en ppm) :
7,56 (d, 1H, aromatique en 5)
7,2 (dd, 1H, aromatique en 7)
6,82 (d, 1H, aromatique en 8)
6,3 (t, $-C\underline{H}=$)
4,22 (mt, $-O-C\underline{H}_2-$ et $-CO_2-C\underline{H}_2-$)
3,37 (td, $-C\underline{H}_2-$ en -3)
1,33 (t, $-C-\underline{H}_3$)

## EXEMPLE 20

On chauffe à reflux pendant 1 heure 30 minutes, 2,3 g de (bromo-2 éthyl)-4 hydroxy-6 dihydro-3,4 2H-benzopyranne, 5,88 g de (diméthoxy-3,4 phényl)-4 pipéridine, 1,5 g d'iodure de potassium dans 70 cm3 de butanone-2.

On filtre le mélange réactionnel sur verre fritté puis on évapore le solvant sous pression réduite (5,2 kPa). Le résidu obtenu est chromatographié sur une colonne de 4,4 cm de diamètre contenant 100 g de gel de silice en éluant par un mélange de dichlorométhane et d'isopropanol (90-10 en volumes) et en recueillant des fractions de 20 cm3. Les fractions comprises entre 200 et 540 cm3 sont concentrées à sec.

Après une première recristallisation dans 30 cm3 d'éthanol et une deuxième recristallisation dans 30 cm3 de méthanol, on obtient 1,2 g d'iodhydrate de (diméthoxy-3,4 phényl)-1 [(hydroxy-6 dihydro-3,4 2H-benzopyran-1 yl-4)-2 éthyl]-4 pipéridine sous forme d'un solide blanc fondant avec décomposition aux environs de 200°C.

Le (bromo-2 éthyl)-4 hydroxy-6 dihydro-3,4 2H-benzopyranne peut être préparé par chauffage pendant 4 heures d'une solution de 3 g de (bromo-2 éthyl)-4 méthoxy-6 dihydro-3,4 2H-benzopyranne dans 30 cm3 d'acide acétique additionné de 30 cm3 d'acide bromhydrique concentré.

On concentre le mélange réactionnel sous pression réduite (5,2 kPa), ajoute de l'ammoniaque concentré puis on extrait à l'acétate d'éthyle. La phase organique séchée sur sulfate de magnésium anhydre est ensuite concentrée à sec. Le résidu obtenu est chromatographié sur une colonne de 4,4 cm de diamètre contenant 100 g de silice et en éluant avec un mélange de cyclohexane et d'acétate d'éthyle (70-30 en volumes). Les fractions comprises entre 220 et 295 cm3 sont concentrées à sec et on obtient 2,3 g de (bromo-2 éthyl)-4 hydroxy-6 dihydro-3,4 2H-benzopyranne sous forme d'une huile jaune pâle.

Spectre de RMN (250 MHz, $CDCl_3$, δ en ppm).

6,65 (mt, 3H, aromatiques)
4,7 (s, -O-$\underline{H}$)
4,16 (mt, -O-C$\underline{H}_2$-)
3,53 (mt, -C$\underline{H}_2$-Br)
3,05 (mt, >CH-)
2,31 et 2,07 (mt, -C$\underline{H}_2$-$CH_2$-Br)
2,14 et 1,75 (mt, -$CH_2$- en 3)

EXEMPLE 21

On opère comme à l'exemple 1, à partir de 2 g de (bromo-2 éthyl)-4 nitro-6 dihydro-3,4 2H-benzopyranne, de 1,8 g de chlorhydrate de (diméthoxy-3,4 phényl)-4 pipéridine et de 0,96 g de carbonate de potassium sec puis de 1,16 g d'iodure de potassium dans 70 $cm^3$ de butanone-2.

Le résidu obtenu est purifié par chromatgraphie sur une colonne de 4,4 cm de diamètre contenant 100 g de gel de silice en utilisant un mélange de dichlorométhane et d'éthanol (96-4 en volumes) comme éluant et en recueillant des fractions de 50 $cm^3$. Les fractions comprises entre 0,25 et 1,1 litre sont concentrées à sec. L'huile obtenue est reprise par 50 $cm^3$ d'éthanol puis additionnée de 1,15 $cm^3$ d'une solution 5,5N d'acide chlorhydrique dans l'isopropanol. Le précipité obtenu (1,4 g) est recristallisé dans 15 cm3 de méthanol. On obtient ainsi 0,9 g de chlorhydrate de [(nitro-6 dihydro-3,4 2H-benzopyran-1 yl-4)-2 éthyl]-1 (diméthoxy-3,4 phényl)-4 pipéridine sous forme d'un solide blanc fondant à 209°C.

Le (bromo-2 éthyl)-4 nitro-6 dihydro-3,4 2H-benzopyranne peut être préparé de la manière suivante :

A une solution refroidie entre 10-15°C de 3 g de (bromo-2 éthyl)-4 dihydro-3,4 2H-benzopyranne dans 8,4 cm3 d'acide acétique, on ajoute goutte à goutte 0,9 cm3 d'acide nitrique concentré (d = 1,40). Après 18 heures à 20°C, on verse le mélange réactionnel sur de la glace puis on extrait par 200 cm3 de dichlorométhane. La phase organique après lavages à l'eau est séchée sur sulfate de magnésium puis concentrée à sec sous pression réduite (5,2 kPa). Le résidu obtenu est chromatographié sur une colonne de 4,4 cm de diamètre contenant 100 g de gel de silice en éluant avec un mélange de cyclohexan et d'acétate d'éthyle (90-10 en volumes). Les fractions comprises entre 630 et 730 cm3 sont concentrées à sec et on obtient ainsi 0,35 g de (bromo-2 éthyl)-4 nitro-6 dihydro-3,4 2H-benzopyranne sous forme d'une huile jaune.

Spectre de RMN (250 MHz, $CDCl_3$, δ en ppm).

8,12 (d, 1H en 5, aromatique)
8,03 (dd, 1H en 7, aromatique)
6,9 (d, 1H en 8, aromatique)
4,32 (mt, 2H, -O-C$\underline{H}_2$-)
3,55 (mt, 2H, -C$\underline{H}_2$-Br)
3,20 (mt, 1H, >C$\underline{H}$-)
2,4 et 2,10 (mt, -C$\underline{H}_2$-$CH_2$Br)
2,20 et 1,87 (mt, -C$\underline{H}_2$- en 3)

EXEMPLE 22

On opère comme à l'exemple 1, à partir de 2 g de (bromo-2 éthyl)-4 diméthyl-2,2 dihydro-3,4 2H-benzopyranne, de 1,92 g de dichlorhydrate de (diméthoxy-3,4 phényl)-4 pipéridine et de 1 g de carbonate de potassium sec puis de 1,23 g d'iodure de potassium dans 70 $cm^3$ de butanone-2.

Le résidue obtenu est purifié par chromatographie sur une colonne de 4,4 cm de diamètre contenant 100 g de gel de silice en utilisant un mélange d'acétate d'éthyle et d'éthanol (90-10 en volumes) comme éluant et en recueillant des fractions de 20 cm3. Les fractions comprises entre 320 et 440 cm3 sont concentrées à sec. On reprend l'huile obtenue par 50 cm3 d'éthanol et ajoute 0,93 cm3 d'une solution 5,5N d'acide chlorhydrique dans l'isopropanol. Les cristaux formés sont ensuite recristallisés dans 23 cm3 d'éthanol et on obtient ainsi 1 g de chlorhydrate de (diméthoxy-3,4 phényl)-1 [(diméthyl-2,2 dihydro-3,4 2H-benzopyran-1 yl-4)-2 éthyl]-4 pipéridine sous forme de cristaux blancs fondant à 220°C.

Le (bromo-2 éthyl)-4 diméthyl-2,2 dihydro-3,4 2H-benzopyranne peut être préparé en opérant comme à l'exemple 1 mais à partir de 7 g de (diméthyl-2,2 dihydro-3,4 2H-benzopyran-1 yl-4)-2 éthanol, de 5,35 g de NN'-carbonyldiimidazole et de 19,8 cm3 de bromure d'allyle dans 50 cm3 d'acétonitrile.

L'huile ainsi obtenue est chromatographiée sur une colonne de 4,4 cm de diamètre contenant 100 g de gel de silice en éluant par le dichlorométhane et en recueillant des fractions de 20 cm3. Les fractions comprises entre 140 et 240 cm3 sont concentrées à sec.

On obtient 7,66 g de (bromo-2 éthyl)-4 diméthyl-2,2 dihydro-3,4 2H-benzopyranne sous forme d'un solide fondant à 72°C.

Le (diméthyl-2,2 dihydro-3,4 2H-benzopyran-1 yl-4)-2 éthanol peut être préparé en opérant comme à l'exemple 1 mais à partir de 2,5 g d'hydrure de lithium et d'aluminium, de 8,2 g de (diméthyl-2,2 dihydro-3,4 2H-benzopyran-1 yl-4)-2 éthanoate d'éthyle dans 180 cm3 de tétrahydrofuranne.

On obtient ainsi 7 g de (diméthyl-2,2 dihydro-3,4 2H-benzopyran-1 yl-4)-2 éthanol sous forme d'un solide blanc fondant aux environs de 67°C.

Le (diméthyl-2,2 dihydro-3,4 2H-benzopyran-1 yl-4)-2 éthanoate d'éthyle peut être préparé en opérant comme à l'exemple 1 mais à partir de 8,4 g de diméthyl-2,2 dihydro-3,4 2H-benzopyran-1 ylidène-4 acétate d'éthyle de 0,42 g de palladium sur charbon (10%) dans 200 cm3 d'éthanol.

On obtient ainsi 8,2 g de (diméthyl-2,2 dihydro-3,4 2H-benzopyran-1 yl-4)-2 éthanoate d'éthyle sous forme d'une huile jaune pâle.

Spectre de RMN (200 MHz, $CDCl_3$, δ en ppm):

7,12 et 6,85 (mt, 4H, aromatiques)
4,2 (q, $-CO_2-C\underline{H}_2-$)
3,37 (mt, >CH-)
3 et 2,38 (dd, $-C\underline{H}_2-CO_2-$)
2 et 1,60 (dd et t, $-CH_2-$ en 3)
1,43 et 1,27 (s, >$C(C\underline{H}_3)_2$)
1,28 (t, $-CH_2-C\underline{H}_3$)

Le diméthyl-2,2 dihydro-3,4 2H-benzopyran-1 ylidène-4 acétate d'éthyle peut être préparé en opérant comme à l'exemple 1, mais à partir de 4,7 g d'hydrure de sodium (80%), de 3,8 g de diéthylphosphonoacétate d'éthyle et de 10 g de diméthyl-2,2 chromannone-4 dans 200 cm3 de diméthoxyéthane.

Le résidu obtenu est chromatographié sur une colonne de 4,4 cm de diamètre contenant 100 g de gel de silice en éluant avec un mélange de cyclohexane et d'acétate d'éthyle (95-5 en volumes) et en recueillant les fractions comprises entre 150 et 510 cm3.

On obtient ainsi 9,5 g d'un mélange d'isomères de diméthyl-2,2 dihydro-3,4 2H-benzopyran-1 ylidène-4 acétate d'éthyle sous forme d'une huile jaune pâle.

Spectre de RMN (250 MHz, $CDCl_3$, δ en ppm).

| | | |
|---|---|---|
| Isomère E (40%) | ) | 7,59 (dd, 1H, aromatique) |
| | ) | 6,41 (t, -CH=) |
| | ) | 3,3 (d, $-CH_2-$ en 3) |
| Isomère Z (40%) | ) | 7,87 (dd, 1H, aromatique) |
| | ) | 5,7 (s, -CH=) |
| | ) | 2,47 (s, $-C\underline{H}_2-$ en 3) |
| Isomère endo (20%) | ) | 5,58 (s, -CH=) |
| | ) | 3,4 (s, $-C\underline{H}_2-CO_2-$) |

La diméthyl-2,2 chromannone-4 peut être préparée selon la technique décrite dans le brevet belge 844 943.

EXEMPLE 23

On opère comme à l'exemple 1, à partir de 2 g de (bromo-2 éthyl)-4 dihydro-3,4 2H-benzopyranne, de 2,3 g de dichlorhydrate de (méthylènedioxy-3,4 phényl)-1 pipérazine et de 2,28 g de carbonate de potassium sec, puis de 1,4 g d'iodure de potassium dans 70 $cm^3$ de butanone-2.

Après chromatographie du résidu sur une colonne de 4,4 cm de diamètre contenant 100 g de gel de silice, en utilisant l'acétate d'éthyle comme éluant et en recueillant des fractions de 30 $cm^3$, on isole par concentration des fractions comprises entre 210 et 360 $cm^3$, une huile que l'on fait cristalliser dans 40 $cm^3$ d'éthanol additionné de 2,4 $cm^3$ d'une solution 5N d'acide chlorhydrique dans l'isopropanol.

On obtient ainsi 2,18 g de cristaux blancs de dichlorhydrate de [(dihydro-3,4 2H-benzopyran-1 yl-4)-2 éthyl]-1 (méthylènedioxy-3,4 phényl)-4 pipérazine dont le point de fusion est voisin de 180°C.

EXEMPLE 24

On opère comme à l'exemple 1, à partir de 2 g de (bromo-2 éthyl)-4 dihydro-3,4 2H-benzopyranne, de 2,43 g de dichlorhydrate de (pipérazinyl)-1)-6 benzodioxanne-1,4 et de 2,28 g de carbonate de potassium sec puis de 3,4 g d'iodure de potassium dans 70 $cm^3$ de butanone-2.

Le résidu obtenu est purifié par chromatographie sur une colonne de 4,4 cm de diamètre contenant 100 g de gel de silice en utilisant 800 $cm^3$ d'acétate d'éthyle comme éluant et en recueillant des fractions de 20 $cm^3$. Les fractions comprises entre 300 et 680 $cm^3$ sont concentrées à sec. L'huile obtenue (1,6 g) est reprise par 20 $cm^3$ d'éthanol puis additionnée de 1,7 $cm^3$ d'une solution 5N d'acide chlorhydrique dans l'isopropanol. On reprend les cristaux obtenus dans 30 cm3 de méthanol et on porte à reflux, filtre à chaud et on fait précipiter par addition de 50 cm3 d'éther éthylique.

On obtient ainsi 0,9 g de dichlorhydrate de {[dihydro-3,4 2H-benzopyran-1 yl-4)-2 éthyl]-4 pipérazinyl-1}-6 benzodioxanne-1,4.

Le dichlorhydrate de (pipérazinyl-1)-6 benzodioxanne-1,4 peut être préparé de la manière suivante :

On dissout 30,2 g de l'amino-6 benzodioxanne-1,4 et 42,84 g du chlorhydrate de bis(chloro-2 éthyl) amine dans 300 cm3 de butanol-2 et on chauffe à reflux pendant 2 heures. A la solution refroidie à 80°C, on ajoute 27,6 g de carbonate de potassium sec et on chauffe à reflux le mélange résultant pendant 18 heures. Après refroidissement, on filtre le précipité formé, lave par l'acétone puis on dissout l'insoluble dans 500 cm3 d'eau, neutralise à la soude concentrée puis extrait par 400 cm3 de dichlorométhane. La phase organique est ensuite séchée sur sulfate de magnésium et après filtration on concentre sous pression réduite (5,2 kPa).

On reprend l'huile obtenue par 50 cm3 d'éthanol et ajoute 100 cm3 d'une solution 5N d'acide chlorhydrique dans l'isopropanol. Le précipité formé est ensuite porté à ébullition dans 1 litre de méthanol. Après refroidissement, on filtre les cristaux formés et on obtient ainsi 36,9 g de dichlorhydrate de (pipérazinyl-1)-6 benzodioxanne-1,4 sous forme d'un solide fondant avec décomposition à 205°C.

EXEMPLE 25

On opère comme à l'exemple 1, à partir de 10 g de (bromo-2 éthyl)-4 dihydro-3,4 2H-benzopyranne, de 9,87 g de (méthoxy-3 nitro-4 phényl)-1 pipérazine, de 5,75 g de carbonate de potassium sec puis de 6,9 g d'iodure de potassium dans 150 cm3 de butanone-2 en chauffant au reflux penant 8 heures.

Le résidu obtenu est purifié par chromatographie sur une colonne de 5 cm de diamètre contenant 400 g de gel de silice en utilisant d'abord comme éluant 1 litre de dichlorométhane pur puis le mélange dichlorométhane-méthanol (97-3 en volumes) et en recueillant des fractions de 125 cm3. Les fractions comprises entre 2 litres et 3,125 litres sont concentrées à sec pour donner 16,1 g d'une huile orangée. Cette huile est reprise par 250 cm3 d'acétone et le chlorhydrate est formé par addition de 16 cm3 d'une solution 5N d'acide chlorhydrique dans l'isopropanol. Après repos 1 heure à 10°C, essorage et lavages avec deux fois 50 cm3 d'acétone puis 50 cm3 d'éther éthylique, on obtient 11,9 g de solide jaune. Ce solide est recristallisé dans 100 cm3 de méthanol bouillant. Après repos 30 minutes à 20°C, les cristaux sont essorés, lavés deux fois avec 10 cm3 de méthanol puis 25 cm3 d'éther éthylique.

On obtient 9,45 g de chlorhydrate de [(dihydro-3,4 2H-benzopyran-1 yl-4)-2 éthyl]-1 (méthoxy-3 nitro-4 phényl)-4 pipérazine sous forme d'un solide jaune fondant à 175°C.

La (méthoxy-3 nitro-4 phényl)-1 pipérazine peut être préparée de la manière suivante :

Un mélange de 25 g de chloro-5 nitro-2 anisole, 55 g de pipérazine anhydre et 20 cm3 de toluène est porté à léger reflux pendant 45 minutes.

Le mélange réactionnel chaud est versé sur 500 cm3 d'eau distillée et 100 cm3 de toluène. Après 30 minutes d'agitation, le mélange réactionnel tiède est essoré et lavé avec deux fois 50 cm3 d'eau puis deux fois 50 cm3 d'éther éthylique.

On obtient 9,87 g de (méthoxy-3 nitro-4 phényl)-1 pipérazine sous forme d'un solide jaune fondant à 135°C.

EXEMPLE 26

On opère comme à l'exemple 1, mais à partir de 1,5 g de (bromo-2 éthyl)-4 dihydro-3,4 2H-benzopyranne, de 1,02 g de benzyl-4 pipéridine et de 0,8 g de carbonate de potassium sec puis de 1 g d'iodure de potassium dans 50 $cm^3$ de butanone-2.

On reprend l'huile obtenue par 30 $cm^3$ d'éthanol et ajou- te 1,6 $cm^3$ d'une solution 2N d'acide chlorhydrique dans l'éthanol.

On obtient ainsi 1,15 g de chlorhydrate de [(dihydro-3,4 2H-benzopyran-1 yl-4)-2 éthyl]-1 benzyl-4 pipéridine sous forme d'un solide blanc fondant à 210°C.

EXEMPLE 27

On opère comme à l'exemple 1, à partir de 1,7 g de (bromo-2 éthyl)-4 dihydro-3,4 2H-benzopyranne, de 1,8 g de (méthyl-4 phénylméthyl)-1 pipérazine et de 1,93 g de carbonate de potassium sec puis de 1,15 g d'iodure de potassium dans 50 $cm^3$ de butanone-2.

Le résidu obtenu est purifié par chromatographie sur une colonne de 4,4 cm de diamètre contenant 75 g de gel de silice en utilisant comme éluant l'acétate d'éthyle. Les fractions comprises entre 700 et 1000 cm3 sont concentrées à sec. L'huile obtenue est reprise par 20 cm3 d'éthanol puis additionnée de 5,5 cm3 d'une solution 2N d'acide chlorhydrique. On obtient 2,05 g de dichlorhydrate de [(dihydro-3,4 2H-benzopyran-1 yl-4)-2 éthyl]-1 (méthyl-4 phénylméthyl)-4 pipérazine sous forme d'un solide blanc dont le point de fusion est de 230°C.

La (méthyl-4 phénylméthyl)-1 pipérazine peut être préparée selon la méthode décrite dans le brevet US 4 421 753.

EXEMPLE 28

On opère comme à l'exemple 1, à partir de 2 g de (bromo-2 éthyl)-4 dihydro-3,4 2H-benzopyranne, de 2,56 g de dichlorhydrate de (diméthoxy-3,4 phénylméthyl)-1 pipérazine et de 2,52 g de carbonate de potassium sec puis de 3 g d'iodure de potassium dans 70 $cm^3$ de butanone-2. L'huile obtenue est purifiée par chromatographie sur une colonne de 4,4 cm de diamètre contenant 100 g de gel de silice en utilisant 900 $cm^3$ d'acétate d'éthyle comme éluant et en recueillant des fractions de 50 $cm^3$. Les fractions comprises entre 700 et 900 $cm^3$ sont concentrées à sec. L'huile obtenue est reprise par 30 $cm^3$ d'éthanol puis additionnée de 3,3 $cm^3$ d'une solution 5N d'acide chlorhydrique dans l'isopropanol. Le précipité filtré conduit à 2,5 g de dichlorhydrate de [(dihydro-3,4 2H-benzopyran-1 yl-4)-2 éthyl]-1 (diméthoxy-3,4 phénylméthyl)-4 pipérazine sous forme d'un solide blanc fondant à 250°C.

Le dichlorhydrate de (diméthoxy-3,4 phénylméthyl)-1 pipérazine peut être préparé selon la demande de brevet japonais 82 093 962.

EXEMPLE 29

On opère comme à l'exemple 1, à partir de 1,31 g de (bromo-2 éthyl)-4 dihydro-3,4 2H-benzopyranne, de 1,28 g de (diméthoxy-3,4 phénylméthyl)-4 pipéridine et de 0,35 g de carbonate de potassium sec puis de 0,9 g d'iodure de potassium dans 70 $cm^3$ de butanone-2.

Le résidu obtenu est purifié par chromatographie sur une colonne de 5,5 cm de diamètre contenant 50 g de gel de silice en utilisant 510 $cm^3$ d'acétate d'éthyle comme éluant et en recueillant des fractions de 30 $cm^3$. Les fractions comprises entre 180 et 510 $cm^3$ sont concentrées à sec. L'huile obtenue est reprise par 20 $cm^3$ d'éthanol puis additionnée de 0,7 $cm^3$ d'une solution 5N d'acide chlorhydrique dans l'isopropanol.

Par addition de 200 $cm^3$ d'éther éthylique, on obtient 1,26 g de chlorhydrate de [(dihydro-3,4 2H-benzopyran-1 yl-4)-2 éthyl]-1 (diméthoxy-3,4 phénylméthyl)-4 pipéridine sous forme d'un solide blanc fondant à 184°C.

La (diméthoxy-3,4 phénylméthyl)-4 pipéridine est préparée par hydrogénation à 20°C sous deux atmosphères, en présence de 0,042 g d'hydroxyde de palladium, de 0,42 g de (diméthoxy-3,4 benzylidène)-4 phénylméthyl-1 pipéridine dans 100 $cm^3$ de méthanol additionné de 0,26 $cm^3$ d'une solution 5N d'acide chlorhydrique dans l'isopropanol. Après filtration et évaporation du solvant sous pression réduite, on isole 0,3 g de (diméthoxy-3,4 phénylméthyl)-4 pipéridine sous forme d'un solide blanc dont le point de fusion est de 76°C.

La (diméthoxy-3,4 benzylidène)-4 phénylméthyl-1 pipéridine peut être préparée de la manière suivante :

A 4,9 g de phénylméthyl-1 pipéridinone-4 dans 100 $cm^3$ de toluène chauffé à 90°C, on ajoute l'ylure de phosphore obtenu par agitation à température ambiante (4 heures), de 16,8 g de bromure de diméthoxy-3,4 phénylméthyltriphénylphosphonium et de 3,82 g de tertiobutylate de potassium dans 50 $cm^3$ de toluène.

Après 3 heures de chauffage, on reprend par 50 $cm^3$ d'acide chlorhydrique 6N. Après neutralisation, la phase aqueuse est extraite par deux fois 400 $cm^3$ de dichlorométhane. La phase organique est ensuite séchée sur sulfate de magnésium puis concentrée à sec sous pression réduite.

Le résidu obtenu est chromatographié sur une colonne de 5,5 cm de diamètre contenant 150 g de gel de silice en utilisant comme éluant un mélange cyclohexane-acétate d'éthyle (50-50 en volume) et en recueillant des fractions de 30 $cm^3$. Les fractions comprises entre 360 et 750 $cm^3$ sont concentrées à sec.

On obtient ainsi 5,6 g de (diméthoxy-3,4 benzylidène)-4 phénylméthyl-1 pipéridine sous forme d'un solide blanc fondant à 91°C.

Le bromure de diméthoxy-3,4 phénylméthyltriphénylphosphonium peut être préparé de la manière suivante :

On chauffe, à 75°C, pendant 1 heure 30 minutes, 11,6 g de diméthoxy-3,4 $\alpha$-bromotoluène [GORDON N-.WALKER et coll., J. Org. Chem, 26, 2740 (1961)] et 13,11 g de triphénylphosphine dans 150 $cm^3$ de diméthylformamide. Après addition de 700 $cm^3$ d'éther éthylique au mélange réactionnel et filtration, on obtient 19,8 g de bromure de diméthoxy-3,4 phénylméthyltriphénylphosphonium sous forme d'un solide blanc fondant à 260°C.

EXEMPLE 30

On opère comme à l'exemple 1, mais à partir de 2 g de (bromo-2 éthyl)-4 dihydro-3,4 2H-benzopyranne, de 2,76 g de pyridyl-4 pipérazine et de 1,37 g d'iodure de potassium dans 70 $cm^3$ de butanone-2.

Le produit brut obtenu est purifié par chromatographie sur une colonne de 4,4 cm de diamètre contenant 50 g de gel de silice en utilisant comme éluant un mélange de toluène et de diéthylamine (95-5 en volumes). Les 600 premiers cm3 sont concentrés à sec. L'huile obtenue est reprise par 20 cm3 déthanol puis additionnée de 1,6 cm3 d'une solutions 5,5N d'acide chlorhydrique dans l'isopropanol. On obtient 0,7 g de cristaux qui sont recristallisés deux fois dans l'éthanol aqueux (97,5-2,5 en volumes) et on isole ainsi 0,41 g de dichlorhydrate de [(dihydro-3,4 2H-benzopyran-1 yl-4)-2 éthyl]-1 pyridyl-4 pipérazine sous forme d'un solide blanc fondant à 250°C.

EXEMPLE 31

On opère comme à l'exemple 1, à partir de 2 g de (bromo-2 éthyl)-4 dihydro-3,4 2H-benzopyranne, de 1,953 g de (pipéridinyl-4)-1 dihydro-1,3 2H-benzimidazolone-2 et de 1,15 g de carbonate de potassium sec puis de 1,38 g d'iodure de potassium dans 65 cm3 de butanone-2.

On extrait le résidu obtenu par 300 cm3 d'éther éthylique, lave par 10 cm3 d'une solution aqueuse (1N) d'hydroxyde de sodium puis à l'eau (20 cm3) et on sèche sur sulfate de magnésium.

Au résidu obtenu solubilisé dans le minimum d'isopropanol, on ajoute une solution chaude de 0,9 g d'acide fumarique dissous dans le minimum d'isopropanol. On abandonne une nuit à 20°C, filtre les cristaux obtenus puis recristallise dans un mélange de méthanol aqueux (50-50 en volumes).

On obtient ainsi 2,55 g de fumarate acide de {[(dihydro-3,4 2H-benzopyran-1 yl-4)-2 éthyl]-1 pipéridinyl-4}-1 dihydro-1,3 2H-benzimidazolone-2 sous forme d'un solide blanc fondant à 260°C.

EXEMPLE 32

On chauffe à reflux pendant 7 heures 45 minutes, 10 g de dichlorhydrate de [(dihydro-3,4 2H-benzopyran-1 yl-4)-2 éthyl]-1 (méthoxy-4 phényl)-4 pipérazine dans 150 cm3 d'une solution d'acide bromhydrique concentré et 150 cm3 d'acide acétique pur. On concentre le mélange réactionnel sous pression réduite (5,2 kPa), on ajoute de l'ammoniaque concentré puis on extrait à l'acétate d'éthyle. La phase organique séchée sur sulfate de magnésium anhydre est ensuite concentrée à sec.

Le résidu obtenu est chromatographié sur une colonne de 3,5 cm de diamètre conteant 250 g de gel de silice en utilisant comme éluant 1,2 litre d'un mélange de dichlorométhane et d'éthanol (96-4 en volumes) et 1 litre d'un mélange de dichlorométhane et d'éthanol (90-10 en volumes). Les fractions comprises entre 1,2 litres et 1,6 litres sont concentrées à sec.

On reprend le résidu obtenu par le minimum d'éthanol et ajoute 8 cm3 d'une solution 5N d'acide chlorhydrique dans l'isopropanol. Le précipité obtenu par addition d'éther éthylique est ensuite recristallisé dans 130 cm3 d'éthanol aqueux (98-2 en volumes). On obtient ainsi 3 g de dichlorhydrate de [(dihydro-3,4 2H-benzopyran-1 yl-4)-2 éthyl]-1 (hydroxy-4 phényl)-4 pipérazine sous forme d'un solide blanc fondant à 200°C avec décomposition.

EXEMPLE 33

On chauffe à 100°C pendant 2 heures, 1,9 g de [(dihydro-3,4 2H-benzopyran-1 yl)-4 éthyl]-1 (méthoxy-3 phényl)-4 pipérazine dans 50 cm3 d'acide bromhydrique concentré. Après refroidissement du mélange réactionnel et addition de 100 cm3 d'acétone, on isole après filtration 1,8 g de bromhydrate de [(dihydro-3,4 2H-benzopyran-1 yl)-4 éthyl]-1 (hydroxy-3 phényl)-4 pipéridine sous forme d'un solide légèrement rose fondant à 240°C.

EXEMPLE 34

13,5 g de [(dihydro-3,4 2H-benzopyran-1 yl-4)-2 éthyl]-1 (nitro-4 phényl)-4 pipérazine dans 360 cm3 d'éthanol additionné de 15 cm3 d'acide chlorhydrique concentré sont hydrogénés à 20°C sous pression atmosphérique, en présence de 1,5 g de palladium sur charbon (10%).

Après filtration, on concentre sous pression réduite (5,2 kPa) jusqu'à début de cristallisation. Les cristaux ainsi formés sont recristallisés dans 200 cm3 d'éthanol.

On obtient ainsi 9,6 g de dichlorhydrate d'(amino-4 phényl)-1 [(dihydro-3,4 2H-benzopyran-1 yl-4)-2 éthyl]-4 pipérazine sous forme d'un solide grisâtre fondant à 210°C avec décomposition.

EXEMPLE 35

7,22 g de [(dihydro-3,4 2H-benzopyran-1 yl-4)-2 éthyl]-1 (nitro-4 phényl)-4 pipéridine dans 300 cm3 d'acide acétique sont hydrogénés à 20°C sous une pression de 2,7 atmosphères en présence de 0,72 g de palladium sur charbon (10%).

Après filtration, on concentre sous pression réduite (5,2 kPa) et on ajoute 150 cm3 de dichlorométhane puis 60 cm3 d'une solution aqueuse 1N d'hydroxyde de sodium. Après extraction, on lave la phase organique par 100 cm3 d'eau puis on sèche sur sulfate de magnésium et concentre à sec. L'huile obtenue est chromatographiée sur une colonne de 4,4 cm de diamètre contenant 100 g de gel de silice en éluant avec un mélange d'acétate d'éthyle et d'éthanol (90-10 en volumes). Les fractions comprises entre 240 et 600 cm3 sont concentrées à sec. L'huile isolée est cristallisée dans 30 cm3 d'éthanol, additionnée de 3,1 cm3 d'une solution 5,5N d'acide chlorhydrique dans l'isopropanol. Après deux recristallisations dans 20 cm3 d'éthanol aqueux (63-37 en volumes), on obtient 2,13 g de chlorhydrate d'(amino-4 phényl)-4 [(dihydro-3,4 2H-benzopyran-1 yl-4)-2 éthyl]-1 pipéridine sous forme d'un solide blanc fondant à 210°C avec décomposition.

EXEMPLE 36

On opère comme à l'exemple 16, à partir de 6,51 g de chlorhydrate de [(dihydro-3,4 2H-benzopyran-1 yl-4)-2 éthyl]-1 (méthoxy-3 nitro-4 phényl)-4 pipérazine dans 160 cm3 d'éthanol additionnés de 6 cm3 d'une solution d'acide chlorhydrique concentré. L'hydrogénation est effectuée à 40°C sous pression atmosphérique en présence de 0,5 g de palladium sur charbon (à 10%).

Le mélange réactionnel est additionné de 20 cm3 d'eau distillée puis filtré. On concentre sous pression réduite (5,2 kPa) jusqu'à début de la cristallisation.

On obtient ainsi 6,5 g d'un solide qui est recristallisé dans 60 cm3 d'éthanol aqueux (5-1 en volumes). On obtient ainsi 6,3 g de dichlorhydrate d'(amino-4 méthoxy-3 phényl)-1 [(dihydro-3,4 2H-benzopyran-1 yl-4)-2 éthyl]-4 pipérazine dihydratée sous forme d'un solide légèrement grisâtre fondant avec décomposition à 200°C.

EXEMPLE 37

5 g de chlorhydrate de [(dihydro-3,4 2H-benzopyran-1 yl-4)-2 éthyl]-1 (nitro-4 benzoyl)-4 pipérazine dans 350 cm3 d'éthanol sont hydrogénés à 20°C sous pression atmosphérique en présence de 0,5 g de palladium sur charbon (10%) et de 10 cm3 d'une solution 5N d'acide chlorhydrique dans l'isopropanol.

Après filtration, on concentre sous pression réduite (5,2 kPa) jusqu'à 50 cm3. Les cristaux formés sont recristallisés dans 40 cm3 d'éthanol.

On obtient ainsi 1,9 g de chlorhydrate d'(amino-4 benzoyl)-1 [(dihydro-3,4 2H-benzopyran-1 yl-4)-2 éthyl]-4 pipérazine sous forme d'un solide blanc fondant à 205°C.

EXEMPLE 38

5,48 g de [(nitro-6 dihydro-2,4 2H-benzopyran-1 yl-4)-2 éthyl]-1 (diméthoxy-3,4 phényl)-4 pipéridine dans 50 cm3 d'éthanol additionné de 11,6 cm3 d'une solution 5,5N d'acide chlorhydrique dans l'isopropanol sont hydrogénés à 50°C sous pression atmosphérique en présence de 0,54 g de palladium sur charbon (10%).

Après filtration on concentre sous pression réduite (5,2 kPa) et extrait le résidu par 150 cm3 de dichlorométhane, neutralise par 15 cm3 d'une solution 1N de soude puis lave à l'eau. La phase organique est ensuite séchée sur sulfate de magnésium puis concentrée sous pression réduite (5,2 kPa). Le résidu obtenu est chromatographié sur une colonne de 4,4 cm de diamètre contenant 100 g de gel de silice en éluant avec un mélange de dichlorométhane et d'isopropanol (90-10 en volumes) et en recueillant les fractions comprises entre 0,36 et 1,475 litre.

On dissout à chaud, l'huile obtenue, dans 125 cm3 d'éthanol, puis cette solution est ajoutée à une solution de 0,85 g d'acide fumarique dans 15 cm3 d'éthanol. On concentre sous pression réduite jusqu'au début de la cristallisation puis on reprend les cristaux formés dans 125 cm3 d'une solution chaude d'éthanol. Après filtration et refroidissement, on ajoute 50 cm3 d'éther éthylique dans le filtrat, élimine le précipité noir formé puis rajoute 100 cm3 d'éther éthylique.

On obtient ainsi 0,38 g de fumarate d'[(amino-6 dihydro-3,4 2H-benzopyran-1 yl-4) éthyl]-1 (diméthoxy-3,4 phényl)-4 pipéridine sous forme d'un solide crème fondant à 198°C.

EXEMPLE 39

A une solution refroidie entre 0 et 5°C de 4,7 g de dichlorhydrate d'(amino-4 phényl)-4 [(dihydro-3,4 2H-benzopyran-1 yl-4)-2 éthyl]-1 pipérazine dans 110 cm3 de dichlorométhane, on ajoute 4,9 cm3 de triéthylamine puis on introduit 1 cm3 de chlorure de l'acide méthane sulfonique. Après 1 heure on reprend le mélange réactionnel avec 80 cm3 d'eau puis on extrait trois fois par 40 cm3 de dichlorométhane. La phase organique lavée avec 10 cm3 de soude (N) puis à l'eau est séchée sur sulfate de magnésium. Après filtration, on concentre à sec sous pression réduite (5,2 kPa). Le résidu obtenu est chromatographié sur une colonne de 4,4 cm de diamètre contenant 100 g de gel de silice en éluant avec un mélange de dichlorométhane et d'éthanol (95-5 en volumes) et en recueillant des fractions de 20 cm3. Les fractions comprises entre 500 et 700 cm3 sont concentrées à sec. Le résidu obtenu est repris dans 100 cm3 de méthanol et additionné de 2,4 cm3 d'une solution 5,5N d'acide chlorhydrique dans l'isopropanol puis porté à l'ébullition.

Après refroidissement, on obtient 1,3 g de chlorhydrate de [(dihydro-3,4 2H-benzopyran-1 yl-4)-2 éthyl]-1 (méthylsulfonamido-4 phényl)-4 pipérazine sous forme d'un solide blanc fondant à 246°C.

EXEMPLE 40

On opère comme à l'exemple 39, mais à partir de 0,37 g d'(amino-4 phényl)-4 [(dihydro-3,4 2H-benzopyran-1 yl-4)-2 éthyl]-1 pipéridine dans 10 cm3 de dichlorométhane, de 0,155 cm3 de triéthylamine puis de 0,087 cm3 de chlorure de l'acide méthanesulfonique.

Le résidu obtenu est chromatographié sur une colonne de 3 cm de diamètre contenant 50 g de gel de silice en éluant avec un mélange de toluène, de diéthylamine et d'éthanol (90 - 5 - 5 en volumes) et en recueillant les fractions comprises entre 180 et 300 cm3. L'huile obtenue est reprise à l'éther éthylique. La phase organique est lavée à l'eau et séchée sur sulfate de magnésium. Après concentration à sec sous pression réduite (5,2 kPa), l'huile isolée cristallise dans 8 cm3 d'éthanol additionné de 0,4 cm3 d'une solution 5,5N d'acide chlorhydrique dans l'isopropanol.

Après une recristallisation dans 5 cm3 de méthanol, on obtient 0,17 g de chlorhydrate de [(dihydro-3,4 2H-benzopyran-1 yl-4)-2 éthyl])-1 (méthylsulfonamido-4 phényl)-4 pipéridine sous forme d'un solide blanc fondant à 240°C.

EXEMPLE 41

On opère comme à l'exemple 39, à partir de 3,8 g de dichlorhydrate d'(amino-4 méthoxy-3 phényl)-1 [(dihydro-3,4 2H-benzopyran-1 yl-4)-2 éthyl]-1 pipérazine dihydratée, de 7 cm3 de triéthylamine et de 2,6 cm3 de chlorure de l'acide méthanesulfonique dans 60 cm3 de trichlorométhane, à -10°C.

Après 1 heure, le mélange réactionnel est concentré sous pression réduite (5,2 kPa). Le résidu est repris par 60 cm3 d'éthanol et 20 cm3 d'une solution 1N d'acide chlorhydrique puis concentré jusqu'à début de la cristallisation.

On obtient 3 g de cristaux que l'on recristallise deux fois dans 50 cm3 d'éthanol aqueux (98-2 en volumes). Après essorage, on obtient 2,4 g de dichlorhydrate de [(dihydro-3,4 2H-benzopyran-1 yl-4)-2 éthyl]-1 (méthylsulfonamido-4 méthoxy-3 phényl)-4 pipérazine sous forme d'un solide blanc fondant à 180°C.

EXEMPLE 42

On opère comme à l'exemple 39, mais à partir de 3 g (d'amino-4 phényl)-4 [(dihydro-3,4 2H-benzopyran-1 yl-4)-2 éthyl]-1 pipéridine dans 66 cm3 de dichlorométhane, de 1,6 cm3 de triéthylamine puis de 1,8 cm3 de chlorure de l'acide méthanesulfonique, pendant 18 heures à 20°C.

L'huile obtenue est reprise par 70 cm3 d'éthanol puis additionnée de 1,5 cm3 d'une solution 5,5N d'acide chlorhydrique dans l'isopropanol. Les cristaux formés sont ensuite recristallisés dans 56 cm3 d'une solution éthanol - eau (50-6 en volumes) et on obtient ainsi 1,4 g de chlorhydrate de [bis(méthylsulfonyl)amino-4 phényl]-4 (dihydro-3,4 2H-benzopyran-1 yl-4)-2 éthyl]-1 pipéridine sous forme d'un solide blanc fondant à 240°C.

EXEMPLE 43

A une solution refroidie à 5°C de 2 g de dichlorhydrate d'(amino-4 phényl)-1 [(dihydro-3,4 2H-benzopyran-1 yl-4)-2 éthyl]-4 pipérazine dans 15 cm3 de trichlorométhane, on ajoute 3 cm3 de chlorure d'acétyle dans 3 cm3 de trichlorométhane.

Après 2 heures, le mélange réactionnel est concentré sous pression réduite (5,2 kPa) et repris dans 10 cm3 d'eau distillée. L'eau est décantée, et l'huile résiduelle est recristallisée dans 15 cm3 d'éthanol aqueux (10-5 en volumes). Après lente cristallisation à 20°C, les cristaux sont lavés trois fois avec 5 cm3 d'éthanol aqueux (5-5 en volumes), puis avec 10 cm3 d'éther éthylique. Après trois recristallisations dans l'éthanol absolu, on obtient 1,25 g d'(acétamido-4 phényl)-1 [dihydro-3,4 2H-benzopyran-1 yl-4)-2 éthyl]-4 pipérazine sous la forme d'un solide légèrement gris fondant à 160°C.

EXEMPLE 44

On opère comme à l'exemple 39, mais à partir de 2,6 g d'(amino-4 benzoyl)-1 [dihydro-3,4 2H-benzopyran-1 yl-4)-2 éthyl]-4 pipérazine, de 1 cm3 de triéthylamine et de 0,61 cm3 de chlorure de l'acide méthanesulfonique dans 86 cm3 de dichlorométhane.

Le résidu obtenu est chromatographié sur une colonne de 4,4 cm de diamètre contenant 100 g de gel de silice en éluant avec un mélange de trichlorométhane, d'isopropanol et de diéthylamine (90 - 5 - 5 en volumes) et en recueillant des fractions de 50 cm3. Les fractions comprises entre 0,55 litre et 1,3 litre sont concentrées à sec.

On reprend le produit obtenu dans le dichlorométhane, lave à l'eau puis on sèche la phase organique sur sulfate de magnésium. Après évaporation du solvant, on obtient un solide qui est dissous dans 50 cm3 d'éthanol bouillant. On ajoute 0,75 cm3 d'une solution 5,5N d'acide chlorhydrique dans l'isopropanol. Après refroidissement et recristallisation, on obtient 1,37 g de chlorhydrate de [(dihydro-3,4 2H-benzopyran-1 yl-4)-2 éthyl]-1 (méthanesulfonamido-4 benzoyl)-4 pipéridine, sous forme d'un solide blanc fondant aux environs de 260°C.

EXEMPLE 45

On opère comme à l'exemple 39, mais à partir de 1,2 g d'[(amino-6 dihydro-3,4 2H-benzopyran-1 yl-4)-2 éthyl]-1 (diméthoxy-3,4 phényl)-4 pipéridine dans 45 cm3 de dichlorométhane, de 0,43 cm3 de triéthylamine et de 0,24 cm3 de chlorure de l'acide méthanesulfonique.

Le résidu obtenu est chromatographié sur une colonne de 4,4 cm de diamètre contenant 100 g de gel de silice en utilisant comme éluant un mélange de dichlorométhane et d'isopropanol (90-10 en volumes) et en recueillant des fractions de 20 cm3. Les fractions comprises entre 500 et 700 cm3 sont concentrées à sec.

Le résidu obtenu est solubilisé dans le dichlorométhane puis additionné de 100 cm3 d'un mélange d'éthanol et de méthanol (50-50 en volumes) et concentré sous pression réduite (5,2 kPa) jusqu'au début de la cristallisation.

On obtient ainsi 0,7 g de [(méthylsulfonamido-6 dihydro-3,4 2H-benzopyran-1 yl-4) éthyl]-1 (diméthoxy-3,4 phényl)-4 pipéridine sous forme d'un solide blanc fondant à 195°C.

EXEMPLE 46

A une solution refroidie entre 0 et 5°C de 6 g de [(dihydro-3,4 2H-benzopyran-1 yl-4)-2 éthyl]-1 pipérazine dans 100 cm3 de dichlorométhane, on ajoute 3,77 cm3 de triéthylamine puis on introduit 4,5 g du chlorure de l'acide paranitrobenzoïque dans 10 cm3 de dichlorométhane.

Après 2 heures à 20°C, on dilue à l'eau distillée, on sépare la phase organique et on lave par 20 cm3 d'une solution 1N de soude, puis à l'eau. On sèche la phase organique sur sulfate de magnésium puis on concentre à sec. Le résidu obtenu est chromatographié sur une colonne de 4,4 cm de diamétre contenant 100 g de gel de silice en éluant avec un mélange de dichlorométhane et d'éthanol (95-5 en volumes). Les fractions comprises entre 150 et 520 cm3 sont concentrées à sec. On reprend le résidu obtenu dans 100 cm3 d'un mélange d'éthanol et de méthanol (50-30 en volumes) et ajoute 3,9 cm3 d'une solution 5,5N d'acide chlorhydrique dans l'isopropanol. Après recristallisation des cristaux obtenus dans 100 cm3 d'une solution d'éthanol aqueux (92-8 en volumes), on obtient 6,8 g de chlorhydrate de [(dihydro-3,4 2H-benzopyran-1 yl-4)-2 éthyl]-1 (nitro-4 benzoyl)-4 pipérazine sous forme d'un solide blanc fondant à 146°C.

La [(dihydro-3,4 2H-benzopyran-1 yl-4) éthyl]-1 pipérazine peut être préparée de la manière suivante :

On opère comme à l'exemple 1 à partir de 9,7 g de (bromo-2 éthyl)-4 dihydro-3,4 2H-benzopyranne de 10,4 g de pipérazine puis de 13,4 g d'iodure de potassium dans 300 cm3 de butanone-2 mais sans adjonction de carbonate de potassium. L'huile obtenue est chromatographiée sur une colonne de 4,4 cm de diamètre contenant 100 g de gel de silice en utilisant comme élutant un mélange de dichlorométhane, d'éthanol et de diéthylamine (80 - 18 - 2 en volumes). Les fractions comprises entre 200 et 500 cm3 sont concentrées à sec.

On obtient ainsi 7,1 g de [dihydro-3,4 2H-benzopyran-1 yl-4)-2 éthyl]-1 pipérazine sous forme d'une huile qui est utilisée telle quelle pour l'étape suivante.

EXEMPLE 47

On opère comme à l'exemple 46 à partir de 3,27 g [dihydro-3,4 2H-benzopyran-1 yl-4)-2 éthyl]-1 pipérazine, de 2,2 cm3 de triéthylamine et de 2,66 g de chlorure de l'acide diméthoxy-3,4 benzoïque dans 50 cm3 de dichlorométhane.

Le résidu obtenu est chromatographié sur une colonne de 4,4 cm de diamètre contenant 100 g de gel de silice en éluant avec l'acétate d'éthyle et en recueillant les fractions comprises entre 610 et 900 cm3. On reprend le résidu obtenu dans 110 cm3 d'éthanol et ajoute 1,5 cm3 d'une solution (5N) d'acide chlorhydrique dans l'isopropanol et on concentre sous pression réduite (5,2 kPa). Les cristaux obtenus sont repris dans 30 cm3 d'un mélange d'isopropanol et de butanone-2 (50-50 en volumes), filtre à chaud un insoluble, puis ajoute 40 cm3 d'éther éthylique dans le filtrat et on obtient ainsi 0,97 g de chlorhydrate de [(dihydro-3,4 2H-benzopyran-1 yl-4)-2 éthyl]-1 (diméthoxy-3,4 benzoyl)-4 pipérazine sous forme d'un solide blanc fondant à 175°C.

La présente invention concerne également les compositions pharmaceutiques constituées par un produit de formule générale (I) sous forme libre ou sous forme de sel d'addition avec un acide pharmaceutiquement acceptable, à l'état pur ou sous forme d'une association avec tout autre produit pharmaceutiquement compatible, pouvant être inerte ou physiologiquement actif. Les compositions selon l'invention peuvent être utilisés par voie orale ou parentérale.

Comme compositions solides pour administration orale peuvent être utilisés des comprimés, des pilules, des poudres ou des granulés. Dans ces compositions, le produit actif selon l'invention (éventuellement associé à un autre produit pharmaceutiquement compatible) est mélangé à un ou plusieurs diluants ou adjuvants inertes, tels que saccharose, lactose ou amidon. Ces compositions peuvent également comprendre des substances autres que les diluants, par exemple un lubrifiant tel que le stéarate de magnésium.

Comme compositions liquides pour administration orale, on peut utiliser les émulsions pharmaceutiquement acceptables, des solutions, des suspensions, des sirops et des élixirs contenant des diluants inertes tels que l'eau ou l'huile de paraffine. Ces compositions peuvent également comprendre des substances autres que les diluants, par exemple des produits mouillants, édulcorants ou aromatisants.

Les compositions stériles pour administration parentérale peuvent être de préférence des solutions aqueuses ou non aqueuses, des suspensions ou des émulsions. Comme solvant ou véhi- cule, on peut employer l'eau, le propylèneglycol, un polyéthylèneglycol, des huiles végétales, en particulier l'huile d'olive, des esters organiques injectables, par exemple l'oléate d'éthyle ou autres solvants organiques convenables. Ces compositions peuvent également contenir des adjuvants, en particulier des agents mouil- lants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans un milieu stérile injectable.

Les compositions pharmaceutiques selon l'invention sont particulièrement utiles en thérapeutique humaine en réduisant les troubles du rythme cardiaque dus à des phénomènes de ré-entrée, traités ou non, dans les traitements consécutifs à l'infarctus du myocarde ainsi que dans les états angineux chroniques et les cardiopathies de type ischemique.

D'une façon générale, le médecin déterminera la posologie qu'il estime la plus appropriée en fonction de l'âge, du poids et des autres facteurs propres au sujet à traiter.

Généralement les doses sont comprises entre 0,25 et 1,5 g par jour, de produit actif par voie orale ou intraveineuse pour un adulte.

L'exemple suivant donné à titre non limitatif, illustre une composition selon l'invention.

EXEMPLE

On prépare des comprimés ayant la composition suivante :

| | | |
|---|---|---|
| - chlorhydrate de [(dihydro-3,4 2H-benzopyran-1 yl-4)-2 éthyl]-1 (diméthoxy-3,4 phényl)-4 pipéridine .............. | | 136,7 mg |
| - lactose ........................................... | | 50 mg |
| - excipient .............................. | q.s.p. | 250 mg |

**Revendications**

**Revendications pour les Etats contractants suivants : AT, BE, CH, DE, FR, GB, IT, LI, LU, NL, SE**

1. Un nouveau dérivé du benzopyranne caractérisé en ce qu'il répond à la formule générale :

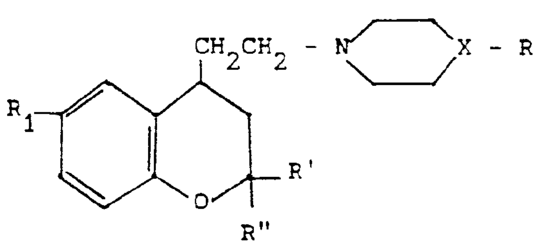

dans laquelle

– $R_1$ représente un atome d'hydrogène ou d'halogène ou un radical hydroxy, alcoyloxy, nitro, amino, alcoylsulfonamido, bis(alcoylsulfonyl)amino, ou acylamino,

– X représente un atome d'azote ou un radical $>$CH-

– R représente un radical de formule générale :

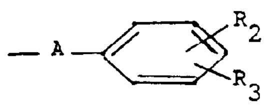

dans laquelle :

A représente une liaison simple, un radical méthylène ou, lorsque X est un atome d'azote A peut représenter un radical carbonyle et $R_2$ et $R_3$ identiques ou différents représentent un atome d'hydrogène ou d'halogène ou un radical hydroxy, alcoyle, alcoyloxy, nitro, amino, alcoylsulfonamido, bis(alcoylsulfonyl-)amino, acylamino, sulfamoyle, ou cyano, ou forment ensemble, lorsqu'ils sont adjacents, un radical méthylènedioxy ou éthylènedioxy, ou bien

R représente un radical pyridyle ou représente un radical 2H-benzimidazolone-2 yle si X représente $>$CH-,

– R′ et R″ sont identiques et représentent des atomes d'hydrogène ou des radicaux alcoyle,

étant entendu que les radicaux alcoyle et acyle cités ci-dessus contiennent 1 à 4 atomes de carbone en chaîne droite ou ramifiée, sous ses formes isomères et leurs mélanges, ainsi que ses sels d'addition avec les acides.

2. Un nouveau dérivé du benzopyranne selon le revendication 1, caractérisé en ce que

– $R_1$ représente un atome d'hydrogène, de chlore ou de fluor, ou un radical hydroxy, méthoxy, nitro, amino ou méthylsulfonamido,

– X représente un atome d'azote ou un radical $>$CH-,

– R représente un radical :

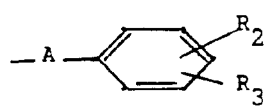

dans laquelle :

A est une liaison simple ou un radical méthylène, ou lorsque X est un atome d'azote, A peut aussi représenter un radical carbonyle et, $R_2$ et $R_3$ identiques ou différents sont situés en position 3 et/ou 4 et représentent un atome d'hydrogène ou de fluor, ou un radical hydroxy, méthyle, méthoxy, nitro, amino, méthylsulfonamido, bis(méthylsulfonyl)amino, acétamido, sulfamoyle ou cyano, ou forment ensemble lorsqu'ils sont adjacents un radical méthylènedioxy ou éthylénedioxy, ou bien

R représente un radical pyridyle ou représente un radical 2H-benzimidazolone-2 yle si X représente $>$CH-,

– R′ et R″ sont identiques et représentent des atomes d'hydrogène ou des radicaux méthyle,

sous ses formes isomères ou leurs mélanges ainsi que ses sels d'addition avec les acides.

3. La [(dihydro-3,4 2H-benzopyran-1 yl-4)-2 éthyl]-1 (diméthoxy-3,4 phényl)-4 pipéridine, ses formes isomères et leurs mélanges, ainsi que ses sels d'addition avec les acides.

4. L'[(amino-6 dihydro-3,4 2H-benzopyran-1 yl-4)-2 éthyl]-1 (diméthoxy-3,4 phényl)-4 pipéridine, ses formes isomères et leurs mélanges, ainsi que ses sels d'addition avec les acides.

5. La [(dihydro-3,4 2H-benzopyran-1 yl-4)-2 éthyl]-1 (méthylsulfonamido-4 phényl)-4 pipéridine, ses formes isomères et leurs mélanges, ainsi que ses sels d'addition avec les acides.

6. La [(dihydro-3,4 2H-benzopyran-1 yl-4)-2 éthyl]-1 (méthylsulfonamido-4 phényl)-4 pipérazine, ses formes isomères et leurs mélanges, ainsi que ses sels d'addition avec les acides.

7. La {[(dihydro-3,4 2H-benzopyran-1 yl-4)-2 éthyl]-1 pipéridinyl-4}-1 dihydro-1,3 2H-benzimidazolone-2, ses formes isomères et leurs mélanges, ainsi que ses sels d'addition avec les acides.

8. Procédé de préparation d'un nouveau dérivé du benzopyranne selon la revendication 1, caractérisé en

ce que l'on fait réagir un produit de formule générale :

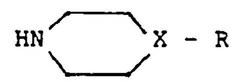

ou son sel, dans laquelle X et R sont définis comme dans la revendication 1, et le cas échéant R étant protégé, sur un dérivé du benzopyranne de formule générale :

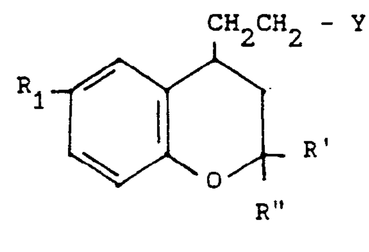

sous ses formes isomères ou leurs mélanges, dans laquelle $R_1$, R′ et R″ sont définis comme dans la revendication 1, le cas échéant $R_1$ étant protégé, et Y représente un atome d'halogène ou un radical alcoylsulfonyloxy ou arylsulfonyloxy, puis élimine le cas échéant les radicaux protecteurs et transforme éventuellement le produit obtenu en un sel d'addition avec un acide.

9. Procédé de préparation selon la revendication 8, caractérisé en ce que l'on met en oeuvre un dérivé du benzopyranne de formule générale :

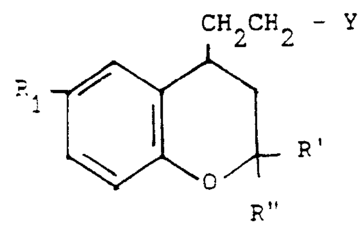

sous ses formes isomères ou leurs mélanges, dans laquelle $R_1$, R′ et R″ sont définis comme dans la revendication 1, et Y est un atome de brome ou de chlore, ou un radical méthylsulfonyloxy ou p.toluènesulfonyloxy.

10. Procédé de préparation d'un nouveau dérivé du benzopyranne selon la revendication 1 pour lequel les radicaux $R_1$, $R_2$ et/ou $R_3$ représentent un radical hydroxy, caractérisé en ce que l'on traite en milieu acide concentré le dérivé correspondant du benzopyranne selon la revendication 1 pour lequel le radical $R_1$, $R_2$ et/ou $R_3$ à transformer représente un radical alcoyloxy, puis transforme éventuellement le produit obtenu en un sel d'addition avec un acide.

11. Procédé de préparation d'un nouveau dérivé du benzopyranne selon la revendication 1, pour lequel les radicaux R, $R_2$ et/ou $R_3$ représentent des radicaux amino, alcoylsulfonamido, bis(alcoylsulfonyl)amino ou acylamino, caractérisé en ce que l'on effectue l'hydrogénation catalytique d'un dérivé du benzopyranne selon la revendication 1 pour lequel le symbole $R_1$, $R_2$ et/ou $R_3$ à transformer représente un radical nitro, puis lorsque l'on veut obtenir un dérivé du benzopyranne pour lequel les radicaux $R_1$, $R_2$ et/ou $R_3$ sont définis comme ci-dessus à l'exception de représenter le radical amino, on transforme le dérivé aminé du benzopyranne obtenu respectivement par sulfonylation ou par acylation, puis transforme éventuellement le produit obtenu en un sel d'addition avec un acide.

12. Procédé de préparation d'un nouveau dérivé du benzopyranne selon la revendication 1, pour lequel le symbole A représente un radical carbonyle, caractérisé en ce que l'on fait agir un acide benzoïque de formule générale :

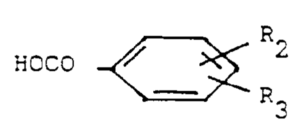

dans laquelle $R_2$ et $R_3$ sont définis comme dans la revendication 1, ou un dérivé réactif de cet acide, sur un dérivé du benzopyranne de formule générale :

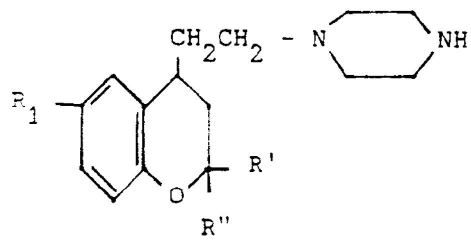

dans laquelle $R_1$, R′ et R″ sont définis comme dans la revendication 1, puis transforme éventuellement le produit obtenu en un sel d'addition avec un acide.

13. Composition pharmaceutique caractérisée en ce qu'elle comprend un dérivé du benzopyranne selon la revendication 1, à l'état pur ou sous forme d'une association avec tout autre diluant ou adjuvant compatible et pharmaceutiquement acceptable.

**Revendications pour les Etats contractants suivants: ES, GR**

1. Procédé de préparation d'un nouveau dérivé du benzopyranne de formule générale :

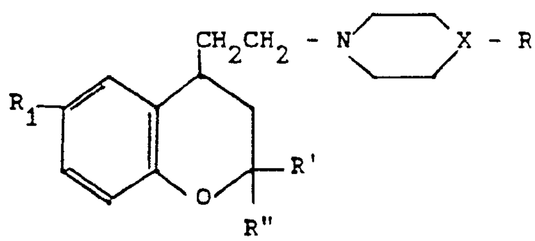

(I)

dans laquelle

- $R_1$, représente un atome d'hydrogène ou d'halogène ou un radical hydroxy, alcoyloxy, nitro, amino, alcoylsulfonamido, bis(alcoylsulfonyl)amino ou acylamino,
- X représente un atome d'azote ou un radical >CH-
- R représente un radical de formule générale :

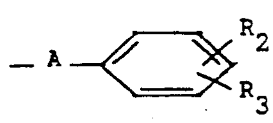

dans laquelle :

A représente une liaison simple, un radical méthylène ou, lorsque X est un atome d'azote A peut représenter un radical carbonyle et $R_2$ et $R_3$ identiques ou différents représentent un atome d'hydrogène ou d'halogène ou un radical hydroxy, alcoyle, alcoyloxy, nitro, amino, alcoylsulfonamido, bis(alcoylsulfonyl-)amino, acylamino, sulfamoyle, ou cyano, ou forment ensemble, lorsqu'ils sont adjacents, un radical méthylènedioxy ou éthylènedioxy, ou bien

R représente un radical pyridyle ou représente un radical 2H-benzimidazolone-2 yle si X représente >CH-,

- R′ et R″ sont identiques et représentent des atomes d'hydrogène ou des radicaux alcoyle,

étant entendu que les radicaux alcoyle et acyle cités ci-dessus contiennent 1 à 4 atomes de carbone en chaîne droite ou ramifiée, sous ses formes isomères et leurs mélanges, ainsi que ses sels d'addition avec les acides, caractérisé en ce que l'on fait réagir un produit de formule générale :

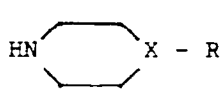

ou son sel, dans laquelle X et R sont définis comme ci-dessus, et le cas échéant R étant protégé, sur un dérivé du benzopyranne de formule générale :

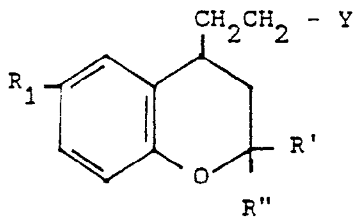

sous ses formes isomères ou leurs mélanges, dans laquelle $R_1$, R′ et R″ sont définis comme ci-dessus, le cas échéant $R_1$ étant protégé, et Y représente un atome d'halogène ou un radical alcoylsulfonyloxy ou arylsulfonyloxy, puis élimine le cas échéant les radicaux protecteurs et transforme éventuellement le produit obtenu en un sel d'addition avec un acide.

2. Procédé de préparation d'un nouveau dérivé du benzopyranne de formule générale (I) pour lequel les radicaux $R_1$, $R_2$ et/ou $R_3$ représentent un radical hydroxy, caractérisé en ce que l'on traite en milieu acide concentré le dérivé correspondant du benzopyranne de formule générale (I) pour lequel le radical $R_1$, $R_2$ et/ou $R_3$ à transformer représente un radical alcoyloxy, puis transforme éventuellement le produit obtenu en un sel d'addition avec un acide.

3. Procédé de préparation d'un nouveau dérivé du benzopyranne de formule générale (I) tel que défini dans la revendication 1 pour lequel les radicaux $R_1$, $R_2$ et/ou $R_3$ représentent des radicaux amino, alcoylsulfonamido, bis(alcoylsulfonyl)amino ou acylamino, caractérisé en ce que l'on effectue l'hydrogénation catalytique d'un dérivé du benzopyranne de formule générale (I) pour lequel les symboles $R_1$, $R_2$ et/ou $R_3$ à transformer représentent un radical nitro, puis lorsque l'on veut obtenir un dérivé du benzopyranne pour lequel les radicaux $R_1$, $R_2$ et/ou $R_3$ sont définis comme ci-dessus à l'exception de représenter le radical amino, on transforme le dérivé aminé du benzopyranne obtenu respectivement par sulfonylation ou par acylation, puis transforme éventuellement le produit obtenu en un sel d'addition avec un acide.

4. Procédé de préparation d'un nouveau dérivé du benzopyranne de formule générale (I) tel que défini dans la revendication 1, pour lequel le symbole A représente un radical carbonyle, caractérisé en ce que l'on fait agir un acide benzoïque de formule générale :

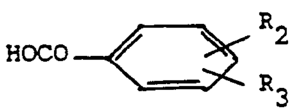

dans laquelle $R_2$ et $R_3$ sont définis comme dans la revendication 1, ou un dérivé réactif de cet acide, sur un dérivé du benzopyranne de formule générale :

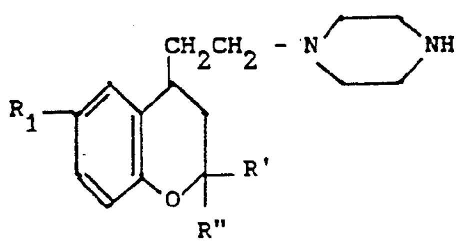

dans laquelle $R_1$, R′ et R″ sont définis comme dans la revendication 1, puis transforme éventuellement le produit obtenu en un sel d'addition avec un acide.

## Patentansprüche

### Patentansprüche für folgende Vertragsstaaten AT, BE, CH, DE, FR, GB IT, LI, LU, NL, SE

1. Neues Benzopyranderivat, dadurch gekennzeichnet, daß es der allgemeinen Formel:

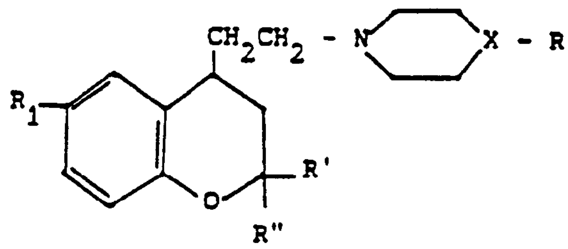

entspricht, in welcher

- $R_1$ ein Wasserstoff- oder Halogenatom oder einen Hydroxy-, Alkyloxy-, Nitro-, Amino-, Alkylsulfonamido-, Bis(alkylsulfonyl)amino- oder Acylaminorest darstellt,
- X ein Stickstoffatom oder einen Rest >CH- darstellt,
- R einen Rest der allgemeinen Formel:

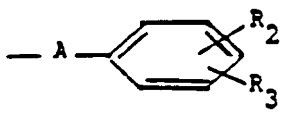

darstellt, in welcher:
A eine Einfachbindung, einen Methylenrest darstellt oder, wenn X ein Stickstoffatom ist, kann A einen Carbonylrest darstellen, und $R_2$ und $R_3$ unabhängig voneinander ein Wasserstoff- oder Halogenatom oder einen Hydroxy-, Alkyl-, Alkyloxy-, Nitro-, Amino-, Alkylsulfonamido-, Bis(alkylsulfonyl)amino-, Acylamino-, Sulfamoyl- oder Cyanorest darstellen oder zusammen, wenn sie benachbart sind, einen Methylendioxy- oder Äthylendioxyrest bilden, oder

R einen Pyridylrest darstellt oder einen 2H-Benzimidazolon-2-ylrest darstellt, wenn X für >CH- steht,

- R′ und R″ gleich sind und Wasserstoffatome oder Alkylreste darstellen, wobei die oben genannten Alkyl- und Acylreste selbstverständlich 1 bis 4 Kohlenstoffatome in gerader oder verzweigter Kette enthalten, in ihren isomeren Formen und deren Mischungen, sowie ihre Säureadditionssalze.

2. Neues Benzopyranderivat nach Anspruch 1, dadurch gekennzeichnet, daß

- $R_1$ ein Wasserstoff-, Chlor- oder Fluoratom oder einen Hydroxy-, Methoxy-, Nitro- Amino- oder Methylsulfonamidorest darstellt,
- X ein Stickstoffatom oder einen Rest >CH- darstellt,
- R einen Rest der allgemeinen Formel:

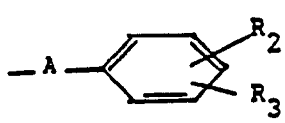

darstellt, in welcher:
A eine Einfachbindung oder ein Methylenrest ist oder, wenn X ein Stickstoffatom ist, kann A auch einen Carbonylrest darstellen, und $R_2$ und $R_3$ befinden sich unabhängig voneinander in 3- und/oder 4-Stellung und stellen ein Wasserstoff- oder Fluoratom oder einen Hydroxy-, Methyl-, Methoxy-, Nitro-, Amino-, Methylsulfonamido-, Bis(methylsulfonyl)amino-, Acetamido-, Sulfamoyl- oder Cyanorest dar oder bilden zusammen, wenn sie benachbart sind, einen Methylendioxy- oder Äthylendioxyrest, oder

R stellt einen Pyridylrest dar oder einen 2H-Benzimidazolon-2-ylrest, wenn X für >CH- steht,

- R′ und R″ gleich sind und stellen Wasserstoffatome oder Methylreste dar, in ihren isomeren Formen und

deren Mischungen, sowie ihre Säureadditionssalze.

3. 1-[2-(3,4-Dihydro-1-2H-benzopyran-4-yl)-äthyl]-4-(3,4-dimethoxyphenyl)-piperidin, seine isomeren Formen und deren Mischungen sowie seine Säureadditionssalze.

4. 1-[2-(6-Amino-3,4-dihydro-1-2H-benzopyran-4-yl)-äthyl]-4-(3,4-dimethoxyphenyl)-piperidin, seine isomeren Formen und deren Mischungen sowie seine Säureadditionssalze.

5. 1-[2-(3,4-Dihydro-1-2H-benzopyran-4-yl)-äthyl]-4-(4-methylsulfonamido-phenyl)-piperidin, seine isomeren Formen und deren Mischungen sowie seine Säureadditionssalze.

6. 1-[2-(3,4-Dihydro-1-2H-benzopyran-4-yl)-äthyl]-4-(4-methylsulfonamido-phenyl)-piperazin, seine isomeren Formen und deren Mischungen sowie seine Säureadditionssalze.

7. 1-{1-[2-(3,4-Dihydro-1-2H-benzopyran-4-yl)-äthyl]-4-piperidiny1}-1,3-dihydro-2-2H-benzimidazolon, seine isomeren Formen und deren Mischungen sowie seine Säureadditionssalze.

8. Verfahren zur Herstellung eines neuen Benzopyranderivats nach Anspruch 1, dadurch gekennzeichnet, daß man eine Verbindung der allgemeinen Formel:

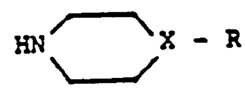

oder ihr Salz, in welcher X und R die im Anspruch 1 angegebene Bedeutung haben und R gegebenenfalls geschützt ist, mit einem Benzopyranderivat der allgemeinen Formel:

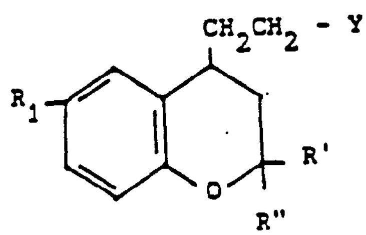

in seinen isomeren Formen oder deren Mischungen, worin $R_1$, R′ und R″ die im Anspruch 1 angegebene Bedeutung haben, $R_1$ gegebenenfalls geschützt ist und Y ein Halogenatom oder einen Alkylsulfonyloxyrest oder Arylsulfonyloxyrest darstellt, reagieren läßt, dann gegebenenfalls die Schutzgruppen entfernt und das erhaltene Produkt gegebenenfalls in ein Säureadditionssalz umwandelt.

9. Herstellungsverfahren nach Anspruch 8, dadurch gekennzeichnet, daß man ein Benzopyranderivat der allgemeinen Formel:

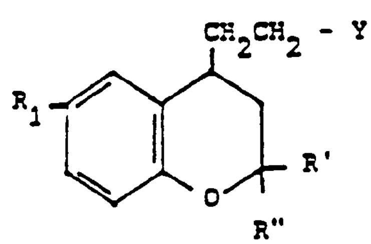

in seinen isomeren Formen oder deren Mischungen einsetzt, worin $R_1$, R′ und R″ die im Anspruch 1 angegebene Bedeutung haben und Y ein Brom- oder Chloratom oder ein Methylsulfonyloxy oder p-Toluolsulfonyloxyrest ist, einsetzt.

10. Verfahren zur Herstellung eines neuen Benzopyranderivats nach Anspruch 1, worin die Reste $R_1$, $R_2$ und/oder $R_3$ einen Hydroxyrest darstellen, dadurch gekennzeichnet, daß man das entsprechende Benzopyranderivat nach Anspruch 1, worin der umzuwandelnde Rest $R_1$, $R_2$ und/oder $R_3$ einen Alkyloxyrest darstellt, in konzentriertem saurem Milieu behandelt und dann gegebenenfalls das erhaltene Produkt in ein Säureadditionssalz umwandelt.

11. Verfahren zur Herstellung eines neuen Benzopyranderivats nach Anspruch 1, worin die Reste $R_1$, $R_2$ und/oder $R_3$ Amino-, Alkylsulfonamido-, Bis(alkylsulfonyl)amino- oder Acylaminoreste darstellen, dadurch gekennzeichnet, daß man die katalytische Hydrierung eines Benzopyranderivats nach Anspruch 1, worin der

umzuwandelnde Rest $R_1$, $R_2$ und/oder $R_3$ einen Nitrorest darstellt, ausführt und dann, wenn man ein Benzopyranderivat, worin die Reste $R_1$, $R_2$ und/oder $R_3$ die obige Bedeutung mit Ausnahme des Aminorestes haben, erhalten will, die erhaltene Aminverbindung von Benzopyran durch Sulfonylierung bzw. durch Acylierung umwandelt und dann gegebenenfalls das erhaltene Produkt in ein Säureadditionssalz umwandelt.

12. Verfahren zur Herstellung eines neuen Benzopyranderivats nach Anspruch 1, worin das Symbol A einen Carbonylrest darstellt, dadurch gekennzeichnet, daß man eine Benzoesäure der allgemeinen Formel:

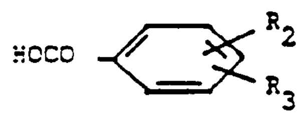

in welcher $R_2$ und $R_3$ die im Anspruch 1 angegebene Bedeutung haben, oder ein reaktionsfähiges Derivat dieser Säure, mit einem Benzopyranderivat der allgemeinen Formel

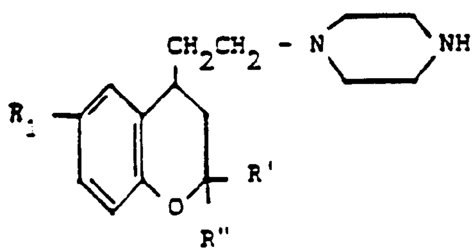

in welcher $R_1$, R′ und R″ die im Anspruch 1 angegebene Bedeutung haben, reagieren läßt und dann gegebenenfalls das erhaltene Produkt in ein Säureadditionssalz umwandelt.

13. Pharmazeutische Massen,dadurch gekennzeichnet, däß sie ein Benzopyranderivat nach Anspruch 1 in reinem Zustand oder in Verbindung mit irgendeinem anderen verträglichen und pharmazeutisch zulässigen Streckmittel oder Zusatzstoff enthalten.

**Patentensprüche für folgende Vertragstaaten: ES, GR**

1. Verfahren zur Herstellung eines neuen Benzopyranderivats der allgemeinen Formel:

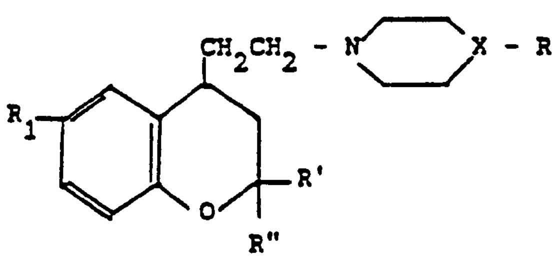

(I)

in welcher

- $R_1$ ein Wasserstoff- oder Halogenatom oder einen Hydroxy-, Alkyloxy-, Nitro-, Amino-, Alkylsulfonamido-, Bis(alkylsulfonyl)amino- oder Acylaminorest darstellt,
- X ein Stickstoffatom oder einen Rest $\geq$CH- darstellt,
- R einen Rest der allgemeinen Formel:

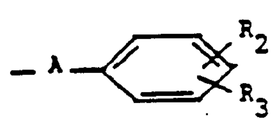

darstellt, in welcher:
A eine Einfachbindung, einen Methylenrest darstellt oder, wenn X ein Stickstoffatom ist, kann A einen Car-

bonylrest darstellen, und $R_2$ und $R_3$ unabhängig voneinander ein Wasserstoff- oder Halogenatom oder einen Hydroxy-, Alkyl-, Alkyloxy-, Nitro-, Amino-, Alkylsulfonamido-, Bis(alkylsulfonyl)amino-, Acylamino-, Sulfamoyl- oder Cyanorest darstellen oder zusammen, wenn sie benachbart sind, einen Methylendioxy- oder Äthylendioxyrest bilden, oder

R einen Pyridylrest darstellt oder einen 2H-Benzimidazolon-2-ylrest darstellt, wenn X für $\geq$ CH- steht,
– R′ und R″ gleich sind und Wasserstoffatome oder Alkylreste darstellen, wobei die oben genannten Alkyl- und Acylreste selbstverständlich 1 bis 4 Kohlenstoffatome in gerader oder verzweigter Kette enthalten, in ihren isomeren Formen und deren Mischungen, sowie ihrer Säureadditionssalze, dadurch gekennzeichnet, daß man eine Verbindung der allgemeinen Formel:

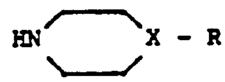

oder ihr Salz, in welcher X und R die im Anspruch 1 angegebene Bedeutung haben und R gegebenenfalls geschützt ist, mit einem Benzopyranderivat der allgemeinen Formel:

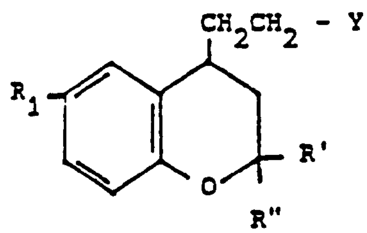

in seinen isomeren Formen oder deren Mischungen, worin $R_1$, R′ und R" die im Anspruch 1 angegebene Bedeutung haben, $R_1$ gegebenenfalls geschützt ist und Y ein Halogenatom oder einen Alkylsulfonyloxyrest oder Arylsulfonyloxyrest darstellt, reagieren läßt, dann gegebenenfalls die Schutzgruppen entfernt und das erhaltene Produkt gegebenenfalls in ein Säureadditionssalz umwandelt.

2. Verfahren zur Herstellung eines neuen Benzopyranderivats der allgemeinen Formel (I), worin die Reste $R_1$, $R_2$ und/oder $R_3$ einen Hydroxyrest darstellen, dadurch gekennzeichnet, daß man das entsprechende Benzopyranderivat der allgemeinen Formel (I), worin der umzuwandelnde Rest $R_1$, $R_2$ und/oder $R_3$ einen Alkyloxyrest darstellt, in konzentriertem saurem Milieu behandelt und dann gegebenenfalls das erhaltene Produkt in ein Säureadditionssalz umwandelt.

3. Verfahren zur Herstellung eines neuen Benzopyranderivats der allgemeinen Formel (I) wie in Anspruch 1 definiert, worin die Reste $R_1$, $R_2$ und/oder $R_3$ Amino-, Alkylsulfonamido-, Bis(alkylsulfonyl)amino- oder Acylaminoreste darstellen, dadurch gekennzeichnet, daß man die katalytische Hydrierung eines Benzopyranderivats der allgemeinen Formel (I), worin der umzuwandelnde Rest $R_1$, $R_2$ und/oder $R_3$ einen Nitrorest darstellt, ausführt und dann, wenn man ein Benzopyranderivat, worin die Reste $R_1$, $R_2$ und/oder $R_3$ die obige Bedeutung mit Ausnahme des Aminorestes haben, erhalten will, die erhaltene Aminverbindung von Benzopyran durch Sulfonylierung bzw. durch Acylierung umwandelt und dann gegebenenfalls das erhaltene Prödukt in ein Säureadditionssalz umwandelt.

4. Verfahren zur Herstellung eines neuen Benzopyranderivats der allgemeinen Formel (I) wie im Anspruch 1 definiert, worin das Symbol A einen Carbonylrest darstellt, dadurch gekennzeichnet, daß man eine Benzoesäure der allgemeinen Formel:

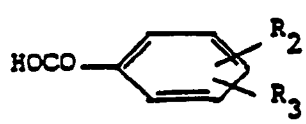

in welcher $R_2$ und $R_3$ die im Anspruch 1 angegebene Bedeutung haben, oder ein reaktionsfähiges Derivat dieser Säure, mit einem Benzopyranderivat der allgemeinen Formel

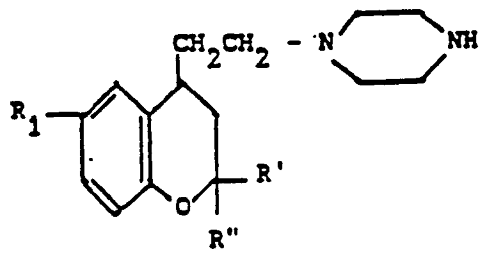

in welcher $R_1$, R′ und R″ die im Anspruch 1 angegebene Bedeutung haben, reagieren läßt und dann gegebenenfalls das erhaltene Produkt in ein Säureadditionssalz umwandelt.

## Claims

**Claims for the following Contracting States: AT, BE, CH, DE, FR, GB, IT, LI, LU, NL, SE**

1. A new benzopyran derivative characterised in that it corresponds to the general formula:

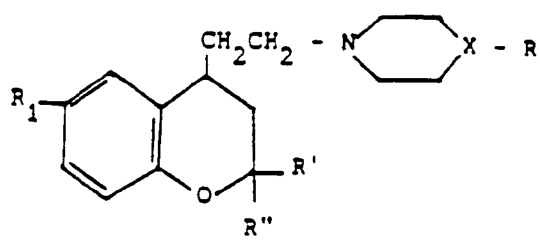

in which

– $R_1$ denotes a hydrogen or halogen atom or a hydroxyl, alkoxy, nitro, amino, alkylsulphonamido, bis(alkylsulphonyl)amino or acylamino radical,

– X denotes a nitrogen atom or a >CH- radical,

– R denotes a radical of general formula:

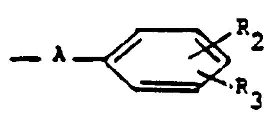

in which:

A denotes a single bond, a methylene radical or, when X is a nitrogen atom A can denote a carbonyl radical, and $R_2$ and $R_3$, which are identical or different, denote a hydrogen or halogen atom or a hydroxyl, alkyl, alkoxy, nitro, amino, alkylsulphonamido, bis(alkylsulphonyl)amino, acylamino, sulphamoyl or cyano radical, or, when they are adjacent, together form a methylenedioxy or ethylenedioxy radical, or else

R denotes a pyridyl radical or denotes a 2(2H)benzimidazolonyl radical if X denotes >CH-, and

– R′ and R″ are identical and denote hydrogen atoms or alkyl radicals, it being understood that the abovementioned alkyl and acyl radicals contain 1 to 4 carbon atoms in a straight or branched chain, in its isomeric forms and mixtures thereof, and its acid addition salts.

2. A new benzopyran derivative according to Claim 1, characterised in that

– $R_1$ denotes a hydrogen, chlorine or fluorine atom, or a hydroxyl, methoxy, nitro, amino or methylsulphonamido radical,

– X denotes a nitrogen atom or a >(CH- radical,

– R denotes a radical:

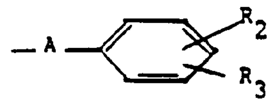

in which:

A is a single bond or a methylene radical or, when X is a nitrogen atom, A can also denote a carbonyl radical, and $R_2$ and $R_3$, which are identical or different, are situated in the 3 and/or 4 position and denote a hydrogen or fluorine atom or a hydroxyl, methyl, methoxy, nitro, amino, methylsulphonamido, bis(methylsulphonyl)amino, acetamido, sulphamoyl or cyano radical, or, when they are adjacent, together form a methylenedioxy or ethylenedioxy radical, or else

R denotes a pyridyl radical or denotes a 2(2H)-benzimidazolonyl radical if X denotes >CH-, and

– R′ and R″ are identical and denote hydrogen atoms or methyl radicals, in its isomeric forms or mixtures thereof, and its acid addition salts.

3. 1-[2-(3,4-Dihydro-1(2H)-benzopyran-4-yl)ethyl]4-(3,4-dimethoxyphenyl)piperidine, its isomeric forms and mixtures thereof, and its acid addition salts.

4. 1-[2-(6-amino-3,4-dihydro-1(2H)-benzopyran-4-yl)-ethyl]-4-(3,4-dimethoxyphenyl)piperidine, its isomeric forms and mixtures thereof, and its acid addition salts.

5. 1-[2-(3,4-Dihydro-1(2H)-benzopyran-4-yl)ethyl]4-(4-methylsulphonamidophenyl)piperidine, its isomeric forms and mixtures thereof, and its acid addition salts.

6. 1-[2-(3,4-Dihydro-1(2H)-benzopyran-4-yl)ethyl]4-(4-methylsulphonamidophenyl)piperazine, its isomeric forms and mixtures thereof, and its acid addition salts.

7. 1-{1-[2-(3,4-Dihydro-1(2H)-benzopyran-4-yl)ethyl]-4-piperidinyl}-1,3-dihydro-2(2H)-benzimidazolone, its isomeric forms and mixtures thereof, and its acid addition salts.

8. A process for the preparation of a new benzopyran derivative according to Claim 1, characterised in that a compound of general formula:

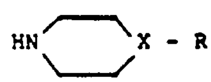

or its salt, in which formula X and R are defined as in Claim 1, and R being protected if appropriate, is reacted with a benzopyran derivative of general formula:

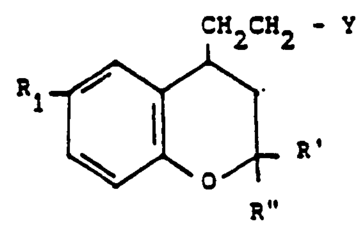

in its isomeric forms or mixtures thereof, in which formula $R_1$, R′ and R″ are defined as in Claim 1, $R_1$ being protected if appropriate, and Y denotes a halogen atom or an alkylsulphonyloxy or arylsulphonyloxy radical, then the protecting radicals are removed if appropriate and the product obtained is optionally converted into an acid addition salt.

9. The preparation process according to Claim 8, characterised in that a benzopyran derivative of general formula:

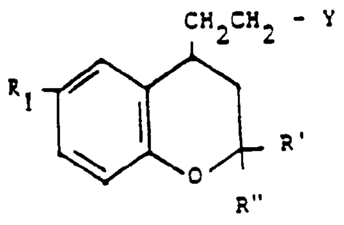

is used in its isomeric forms or mixtures thereof, in which formula $R_1$, R′ and R″ are defined as in Claim 1, and Y is a bromine or chlorine atom, or a methylsulphonyloxy or p-toluenesulphonyloxy radical.

10. A process for the preparation of a new benzopyran derivative according to Claim 1, in which the radicals $R_1$, $R_2$ and/or $R_3$ denote a hydroxyl radical, characterised in that the corresponding benzopyran derivative according to Claim 1, in which the radical $R_1$, $R_2$ and/or $R_3$ to be converted denotes an alkoxy radical, is treated in a concentrated acid medium, and the product obtained is then optionally converted into an acid addition salt.

11. A process for the preparation of a new benzopyran derivative according to Claim 1, in which the radicals $R_1$, $R_2$ and/or $R_3$ denote amino, alkylsulphonamido, bis(alkylsulphonyl)amino or acylamino radicals, characterised in that catalytic hydrogenation of a benzopyran derivative according to Claim 1, in which the radical $R_1$, $R_2$ and/or $R_3$ to be converted denotes a nitro radical, is performed, and then, when the desired product to be obtained is a benzopyran derivative in which the radicals $R_1$, $R_2$ and/or $R_3$ are defined as above except for denoting an amino radical, the benzopyran amine derivative obtained is converted by sulphonylation or acylation respectively, and the product obtained is then optionally converted into an acid addition salt.

12. A process for the preparation of a new benzopyran derivative according to Claim 1, in which the symbol A denotes a carbonyl radical, characterised in that a benzoic acid of general formula:

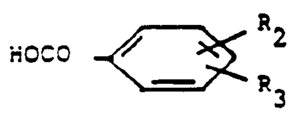

in which $R_2$ and $R_3$ are defined as in Claim 1, or a reactive derivative of this acid, is reacted with a benzopyran derivative of general formula:

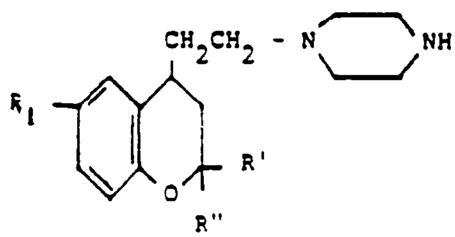

in which $R_1$, R′ and R″ are defined as in Claim 1, and the product obtained is then optionally converted into an acid addition salt.

13. A pharmaceutical composition, characterised in that it comprises a benzopyran derivative according to Claim 1, in the pure state or in the form of a combination with any other compatible and pharmaceutically acceptable diluent or adjuvant.

**Claims for the following Contracting States: ES, GR**

1. A process for the preparation of a new benzopyran derivative of general formula:

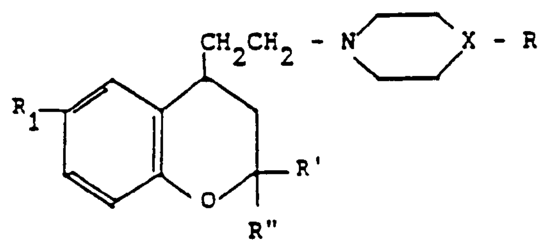

(I)

in which

– $R_1$ denotes a hydrogen or halogen atom or a hydroxyl, alkoxy, nitro, amino, alkylsulphonamido, bis(alkylsulphonyl)amino or acylamino radical,

– X denotes a nitrogen atom or a > CH- radical,

– R denotes a radical of general formula:

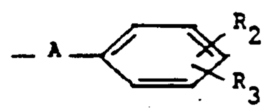

in which:

A denotes a single bond, a methylene radical or, when X is a nitrogen atom A can denote a carbonyl radical, and $R_2$ and $R_3$, which are identical or different, denote a hydrogen or halogen atom or a hydroxyl, alkyl, alkoxy, nitro, amino, alkylsulphonamido, bis(alkylsulphonyl)amino, acylamino, sulphamoyl or cyano radical, or, when they are adjacent, together form a methylenedioxy or ethylenedioxy radical, or else

R denotes a pyridyl radical or denotes a 2(2H)benzimidazolonyl radical if X denotes > CH-, and

– R′ and R″ are identical and denote hydrogen atoms or alkyl radicals,

it being understood that the abovementioned alkyl and acyl radicals contain 1 to 4 carbon atoms in a straight or branched chain, in its isomeric forms and mixtures thereof, and its acid addition salts, characterised in that a compound of general formula:

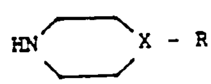

or its salt, in which formula X and R are defined as above, and R being protected if appropriate, is reacted with a benzopyran derivative of general formula:

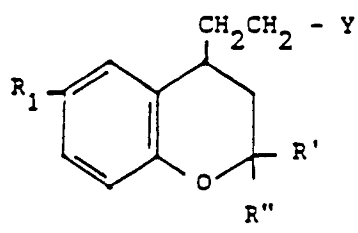

in its isomeric forms or mixtures thereof, in which formula $R_1$, R′ and R″ are defined as above, $R_1$ being protected if appropriate, and Y denotes a halogen atom or an alkyl-sulphonyloxy or arylsulphonyloxy radical, then the protecting radicals are removed if appropriate and the product obtained is optionally converted into an acid addition salt.

2. A process for the preparation of a new benzopyran derivative of general formula (I), in which the radicals $R_1$, $R_2$ and/or $R_3$ denote a hydroxyl radical, characterised in that the corresponding benzopyran derivative of general formula (I), in which the radical $R_1$, $R_2$ and/or $R_3$ to be converted denotes an alkoxy radical, is treated in a concentrated acid medium, and the product obtained is then optionally converted into an acid addition salt.

3. A process for the preparation of a new benzopyran derivative of general formula (I) as defined in claim 1, in which the radicals $R_1$, $R_2$ and/or $R_3$ denote amino, alkylsulphonamido, bis(alkylsulphonyl)amino or

acylamino radicals, characterised in that catalytic hydrogenation of a benzopyran derivative of general formula (I), in which the radicals $R_1$, $R_2$ and/or $R_3$ to be converted denote a nitro radical, is performed and then, when the desired product to be obtained is a benzopyran derivative in which the radicals $R_1$, $R_2$ and/or $R_3$ are defined as above except for denoting an amino radical, the benzopyran amine derivative obtained is converted by sulphonylation or acylation respectively, and the product obtained is then optionally converted into an acid addition salt.

4. A process for the preparation of a new benzopyran derivative of general formula (I) as defined in claim 1, in which the symbol A denotes a carbonyl radical, characterised in that a benzoic acid of general formula:

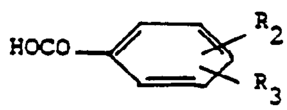

in which $R_2$ and $R_3$ are defined as in claim 1, or a reactive derivative of this acid, is reacted with a benzopyran derivative of general formula:

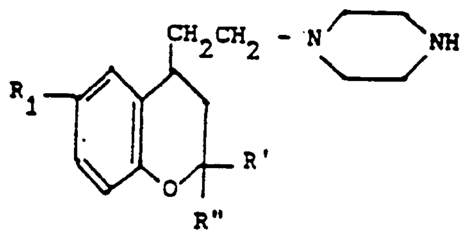

in which $R_1$, R′ and R″ are defined as in claim 1, and the product obtained is then optionally converted into an acid addition salt.